# EUROPEAN PATENT APPLICATION

(11) **EP 1 795 223 A2**
(43) Date of publication of application: **13.06.2007**
(21) Application number: 07100204.2
(22) Date of filing: 25.11.2003
(51) Int. Cl.: A61M 25/01, A61B 1/31, A61B 17/12, A61M 29/00

(54) **Catheter drive**

(30) Priority: 25.11.2002 US 303064
(62) Divisional of application: 03773980.2
(71) Applicant: F.D. Cardio Ltd., 31905 Haifa (IL)
(72) Inventor: Chermoni, Israel, 34679, Haifa (IL)
(74) Representative: van Westenbrugge, Andries

(57) **Abstract**

A catheter (500) for use in a blood vessel, comprising an elongate body having an axis, a lumen (506) along said axis, a proximal opening at one end, connected to the lumen and a front tip at a distal end of the body; and an elongate hydraulic fluid column in said lumen and adapted to apply a pushing force to said front tip in a distal direction, said force being applied at an application point. Optionally, the body comprises two tubes (502,504), one or which extends in response to the pushing force.

## Description

### RELATED APPLICATIONS

This application is a continuation-in-part of US 10/303,064 filed on November 25, 2002, the disclosure of which is incorporated herein by reference.

### FIELD OF THE INVENTION

The present invention relates to devices and methods for catheter navigation.

### BACKGROUND OF THE INVENTION

Blood vessels can suffer from various diseases, in particular arteriosclerosis, in which obstructions form in a lumen of a blood vessel, narrowing or clogging it. Emboli can also cause clogging of blood vessels. A common treatment method for narrowing is inserting a catheter with a balloon at its end to a clogged portion of the blood vessel, inflating the balloon and possibly leaving a stent at the clogged location, to keep the blood vessel open.

In some implementations, a guide wire is brought to the narrowed location, and then the catheter is pushed over the guide wire. If the catheter is soft, helping it negotiating curves, it may be difficult to convey the pushing force along the catheter from outside the body to the tip. If the catheter is stiffer, it may be less able to negotiate sharp curves. In either case, excessive pushing forces may damage the blood vessels.

One solution suggested in the art is providing a catheter with varying levels of stiffness along its length - stiff at its base and soft at its end.

In colonoscopy, US patent 6,485,409, the disclosure of which is incorporated herein by reference, suggests a colonoscope which is advanced by air or liquid pressure. This solution is apparently not suitable for catheters used in blood vessels, especially in narrow diameter blood vessels, such as cardiac arteries and brain arteries, for example due to size considerations and geometry of surfaces tat would contact blood.

### SUMMARY OF THE INVENTION

A broad aspect of some embodiments of the invention relates to a catheter advancing mechanism in which force is applied, via a hydraulic mechanism, near a distal tip of a catheter. In an exemplary embodiment of the invention, the applied force causes the distal tip of the catheter to pull along with it at least a part of the catheter proximal (e.g., further from the tip) of where the force is applied. In another exemplary embodiment of the invention, the applied force pushes a part of the catheter, starting at a point well inside the body, relative to a second part of the catheter which extends to outside of the body and does not move.

In an exemplary embodiment of the invention, the catheter comprises at least two tubes inside the body, an inner tube and an outer tube enclosing at least part of the inner tube. In an exemplary embodiment of the invention, the tips pulls an inner tube section of the catheter out of an outer tube section of the catheter. In an alternative embodiment, the tip pulls the outer tube section of the catheter along an inner tube section of the catheter.

In an embodiment where the outer tube is pulled, a significant length of moving catheter body may be in contact with the enclosing blood vessel (or optional delivery sheath). In an embodiment where the inner tube is pulled, a shorter moving length is generally in contact with the sheath and/or the enclosing blood vessel.

In an exemplary embodiment of the invention, the force is applied using fluid pressure which is optionally applied from outside the body. Optionally, a sliding fluid seal is provided between the outer and inner tubes. The fluid may be provided, for example, in the inner tube, or between the inner tube and the outer tube. Optionally, the same fluid source and path is used for advancing and for balloon inflation. Optionally, a valve is provided to release pressure and prevent over pressuring of the fluid.

Optionally, additionally to fluid pressure, a solid mechanical element force may be used for advancing. For example, a stylet may be inserted through the inner tube to advance the tip. Optionally, such a stylet is used to apply vibration and/or tapping to the tip.

Optionally, a guide wire is provided for the catheter to ride on. In some embodiments the guide wire passes through the fluid path. In others, it does not.

In an exemplary embodiment of the invention, the catheter portion which extends is too soft to be reliably pushed from outside the body along a guide wire to blocked coronaries. However, it is stiff enough to support pushing from a small distance and/or pulling, inside the body. Optionally, the extension mechanism allows a relatively smaller diameter catheter to be used.

In an exemplary embodiment of the invention, the force is applied very close to the tip, for example at a distal part of a balloon portion of the catheter, or less than 5 cm, for example, between 0 and 15 mm, less than 70 mm, less than 30 mm or less than 10 mm, from a proximal part of the balloon portion. In other embodiments, a greater offset is provided between the catheter tip and the location of application of the force, for example, less than 40 cm or less than 30 cm. In an exemplary embodiment of the invention, the offset of the force application point is minimized to allow a relatively rigid catheter portion to reach near a working area (e.g., the aorta) and from there push forward a relatively soft portion (e.g., to a blocked coronary.

Optionally, the catheter includes a stop that prevents over-extension of the catheter tip. Alternatively or additionally, the catheter includes a lock, outside the body, which (optionally selectively) prevents relative motion of the two tubes, or places a limit on such motion, for example, a limit on amount of motion and/or a limit on speed of motion.

In an exemplary embodiment of the invention, a stop wire is provided attached to the moving tube and extending out of a catheter base, and including a brake section selectively movable on the wire. In one example, the distance between the brake section and the catheter base determines an allowed extension. Optionally, the brake is an acceleration brake which resists sudden motion more than slow motion.

An aspect of some embodiments of the invention relates to a balloon catheter having a seal attached thereto, such that the seal can seal fluid flow between the balloon catheter and a guiding catheter, optionally without making any change in the guiding catheter from standard guiding catheters. Optionally, fluid is provided between the balloon catheter and the guiding catheter, to advance the balloon catheter. Optionally, the seal is adaptive and can seal the balloon catheter to a range of guiding catheter inner diameters.

An aspect of some embodiments of the invention relates to a mechanically actuated valve for a catheter. In an exemplary embodiment of the invention, fluid pressure is provided along a lumen of the catheter, and a valve selects whether the fluid will be allowed to apply pressure to a first location and/or to a second location. In one example, the fluid expands a balloon at a first location and applies force to advance a catheter tip, at a second location.

Various valve configurations may be provided. In an exemplary embodiment of the invention, a wire is pulled to remove a blocking element from a lumen of a balloon. When not pulled, the blocking element serves as a base against which advancing force is applied. Alternatively or additionally, a wire is rotated to turn a blocking element so that an aperture therein matches a lumen to which pressure is to be applied.

An aspect of some embodiments of the invention relates to a catheter in which a tip of the catheter advances and lengths the catheter thereby. This lengthening is supported by a section of tube that is in a collapsed configuration outside the body, for example, folded axially, wound in a spiral or folded accordion style. In an exemplary embodiment of the invention, the section of tube is adapted to come in contact with blood. Alternatively or additionally, the section of tube serves as a lumen for expanding a balloon.

There is thus provided in accordance with an exemplary embodiment of the invention, a catheter for use in a blood vessel, comprising:
an elongate body having an axis, a lumen along said axis, a proximal opening at one end, connected to the lumen and a front tip at a distal end of the body; and
an elongate hydraulic fluid column in said lumen and adapted to apply a pushing force to said front tip in a distal direction, said force being applied at an application point. Optionally, said application point is nearer said front tip than said proximal opening. Alternatively or additionally, said proximal opening is adapted to be outside a human body, when the catheter is in use.

In an exemplary embodiment of the invention, said catheter is configured so that said liquid material does not drain into said blood vessel. In an exemplary embodiment of the invention, said column is adapted to be advanced from outside a body.

In an exemplary embodiment of the invention, said body comprises a collapsed tube which extends from said tip to outside of said body and which said pushing force extends collapsed tube.

In an exemplary embodiment of the invention, said tip pulls along a portion of said catheter, having a length of at least 5 times a diameter of the catheter, said length being pulled by said tip when pushing force is applied to said tip.

In an exemplary embodiment of the invention, said body comprises a first, inner, tube and a second, outer tube, said tubes at least partially axially overlapping, wherein said pushing force extends one tube relative to the other tube. Optionally, said tip pulls at least a portion of said one tube with it when pushing force is applied to said tip. Optionally, said pulled section is too soft to be reliably pushed a distance of more than 500 mm in a human body, when the catheter is in use.

In an exemplary embodiment of the invention, said tip pulls along a tube other than said tubes when pushing force is applied to said tip.

In an exemplary embodiment of the invention, at least a portion of said one tube is adapted to be stored outside a human body when the catheter is in use and extends out of a catheter base of said catheter.

In an exemplary embodiment of the invention, at least a portion of said one tube is adapted to be stored outside a human body, when the catheter is in use, in a configuration having a shortened axial dimension.

In an exemplary embodiment of the invention, said inner tube extends when said force is applied.

In an exemplary embodiment of the invention, said outer tube extends when said force is applied.

In an exemplary embodiment of the invention, only one of said inner and said outer tubes substantially extends when said force is applied.

In an exemplary embodiment of the invention, said fluid column is carried between said two tubes.

In an exemplary embodiment of the invention, said fluid column is carried within the inner tube.

In an exemplary embodiment of the invention, the catheter comprises a tool attached at said tip. Optionally, said tool comprises a balloon attached at said tip.

In an exemplary embodiment of the invention, the catheter comprises a separate tube with a lumen for inflating said balloon. Alternatively or additionally, said balloon is attached to a metallic inflation tube. Alternatively, said inner tube serves as a lumen for inflating said balloon. Optionally, said inner tube serves as a lumen for inflating said balloon and not for said fluid column.

In an exemplary embodiment of the invention, said balloon is inflated via a lumen which carries said fluid column. Optionally, said balloon is inflated using a higher pressure than used for extending said catheter. Alternatively or additionally, the catheter comprises a valve at said balloon for selectively allowing liquid flow into said balloon. Optionally, said valve is a pressure sensitive valve. Alternatively, said valve is an externally actuated valve. Optionally, said valve is a stop valve in which a block is retracted from a port to said balloon to allow fluid under pressure to enter the balloon. Alternatively, said valve is a rotating stop valve having at least two configurations, and in which a block is rotated from one configuration to a second one of said configurations to selectively seal or not seal a port to said balloon.

In an exemplary embodiment of the invention, said balloon inflation tube is adapted to be stored outside a human body, when the catheter is in use. Optionally, said tube is stored in an axially collapsed state.

In an exemplary embodiment of the invention, said tube is adapted to extend at least 50 mm.

In an exemplary embodiment of the invention, said one tube is adapted to extend at least 150 mm.

In an exemplary embodiment of the invention, said one tube is adapted to extend at least 250 mm.

In an exemplary embodiment of the invention, said one tube is adapted to extend no more than 500 mm.

In an exemplary embodiment of the invention, the catheter comprises at least one stop which prevents relative motion between the two tubes greater than a pre-set distance.

In an exemplary embodiment of the invention, at least one of said at least one stop is outside of said body.

In an exemplary embodiment of the invention, at least one of said at least one stop is not in contact with said fluid.

In an exemplary embodiment of the invention, said at least one stop comprises a wire extending out of said catheter and at least one movable brake section mounted on said wire.

In an exemplary embodiment of the invention, said stop, when engaged, prevents liquid flow therethrough.

In an exemplary embodiment of the invention, said stop, when engaged, does not prevent liquid flow therethrough.

In an exemplary embodiment of the invention, said stop, is located within 50 mm of a proximal end of the extending tube.

In an exemplary embodiment of the invention, said stop, is located at a distance of at least 50 mm from a proximal end of the extending tube.

In an exemplary embodiment of the invention, when said tube is fully extended, said stop is located at a distal end of the non-extending tube.

In an exemplary embodiment of the invention, when said tube is fully extended, said stop is located at a position spaced less than 50 mm from a distal end of the non-extending tube.

In an exemplary embodiment of the invention, the catheter comprises a plurality of axially spaced stops.

In an exemplary embodiment of the invention, said stop is an element axially shorter than 5 mm. Alternatively, said stop is an element axially longer than 5 mm.

In an exemplary embodiment of the invention, the catheter comprises at least one seal between said tubes. Optionally, said at least one seal is adapted for a particular outer tube inner diameter. Alternatively, said at least one seal is adapted for a range of outer tube inner diameters.

Optionally, said at least one seal comprises a plurality of axial spaced seals. Alternatively, said at least one seal comprises only a single seal.

In an exemplary embodiment of the invention, said at least one seal acts as a stop for preventing over-extension of said one tube.

In an exemplary embodiment of the invention, the catheter comprises an extension limiter which prevents steps of extension greater than a pre-set distance. Optionally, said pre-set extension step limitation is user-settable.

In an exemplary embodiment of the invention, the catheter comprises a lock configured to selectively lock said inner tube to said outer tube and preventing motion.

In an exemplary embodiment of the invention, the catheter comprises a lock configured to selectively couple said other tube to said body.

In an exemplary embodiment of the invention, the catheter comprises a pressure valve configured to release pressure of said working fluid above a certain liquid pressure.

In an exemplary embodiment of the invention, the catheter comprises a controller configured to control extension of said one tube. Optionally, said controller is adapted to extend said tube by a controlled amount. Alternatively or additionally, said controller is adapted to extend said tube by setting a pressure level to be achieved in said liquid. Alternatively or additionally, said controller is adapted to advance said catheter. Alternatively or additionally, said controller is adapted to synchronize a locking of said catheter with inflation of a balloon portion of said catheter. Alternatively or additionally, said controller is adapted to retract said tube relative to said catheter. Optionally, said controller is adapted to synchronize said retraction with advancing of said catheter.

In an exemplary embodiment of the invention, the catheter comprises a guiding sheath surrounding said tubes.

In an exemplary embodiment of the invention, the catheter comprises a guide wire, wherein said catheter is adapted to ride on said guide wire. Optionally, said catheter is configured so that said guide wire passes through said inner tube to outside a human body, when the catheter is in use. Alternatively, said catheter is configured so that said guide wire passes between said inner tube and said outside tube to outside a human body, when the catheter is in use. Alternatively, said catheter is configured so that said guide wire passes outside of said outside tube to outside a human body, when the catheter is in use. Alternatively or additionally, said catheter is configured so that said guide wire passes outside of a guiding sheath to outside a human body, when the catheter is in use.

In an exemplary embodiment of the invention, the catheter comprises a balloon at said tip. Optionally, said guide wire passes through an inflation lumen of said balloon. Alternatively, said guide wire has a proximal exit from said balloon adjacent said balloon. Optionally, said balloon has a thick base from which said guide wire exits.

In an exemplary embodiment of the invention, said exit is less than 20 mm from said balloon.

In an exemplary embodiment of the invention, said guide wire passes within an inflation lumen of said balloon.

In an exemplary embodiment of the invention, said guide wire exits said catheter from said extending tube at a point distal from a most distal point of said non-extending tube.

In an exemplary embodiment of the invention, said guide wire exits said catheter from said extending tube at a point proximal to a most distal point of said non-extending tube.

In an exemplary embodiment of the invention, said guide wire passes through a seal between the two tubes.

In an exemplary embodiment of the invention, said guide wire passes a through a liquid path of said column in said catheter.

In an exemplary embodiment of the invention, said guide wire passes only outside of a liquid path of said column in said catheter.

In an exemplary embodiment of the invention, said inner tube comprises a standard balloon catheter, not manufactured for fluid control and wherein said liquid is carried between said outer tube and said standard balloon catheter.

In an exemplary embodiment of the invention, said inner tube comprises a standard balloon catheter having an adjustable seal mounted thereon, and wherein said liquid is carried between said outer tube and said standard balloon catheter. Optionally, is a guiding catheter.

In an exemplary embodiment of the invention, said outer tube has an outer diameter of less than 3 mm.

In an exemplary embodiment of the invention, said outer tube has an outer diameter of less than 2 mm.

In an exemplary embodiment of the invention, said outer tube has an outer diameter of less than 1 mm.

In an exemplary embodiment of the invention, said inner tube has an outer diameter of less than 1.5 mm.

In an exemplary embodiment of the invention, said inner tube has an outer diameter of less than 0.5 mm.

In an exemplary embodiment of the invention, said application point is less than 500 mm from a most distal point of said catheter.

In an exemplary embodiment of the invention, said application point is less than 350 mm from a most distal point of said catheter.

In an exemplary embodiment of the invention, said application point is less than 70 mm from a most distal point of said catheter.

In an exemplary embodiment of the invention, the catheter comprises an offset element between said application point and said tip, which application point conveys said force from said column towards said tip.

In an exemplary embodiment of the invention, the catheter comprises a push wire adapted to apply a second force to said tip. Optionally, said push wire applies said second force at a substantially same axial position as said application point. Alternatively or additionally, the catheter comprises a controller configured to allow a short advance of said wire, suitable for passing a narrowing in a blood vessel.

In an exemplary embodiment of the invention, the catheter comprises a base hub adapted to remain outside a human body, when the catheter is in use. Optionally, said base hub has only a single port for liquid pressure. Alternatively, said base hub has a plurality of ports for liquid pressure. Optionally, at least one of said ports has a cover adapted to remain closed when fluid inside said port is at 5 atmospheres of pressure or more.

In an exemplary embodiment of the invention, said base hub comprises a pressure release valve. Alternatively or additionally, said base hub comprises a port for a guide wire. Alternatively or additionally, said base hub comprises a port for a pushing wire. Alternatively or additionally, said base hub comprises a port for a valve control wire. Alternatively or additionally, said base hub comprises a port for an extension restricting wire. Optionally, said port is configured to lock said wire when said base is pressurized above a pre-set pressure value.

In an exemplary embodiment of the invention, said base hub comprises a selector configured for selecting which of a plurality of lumens of the catheter fluid pressure will be coupled to.

In an exemplary embodiment of the invention, said base hub comprises a closable opening suitable for selectable user access to a lumen of the catheter through the door. Optionally, said opening is adapted to be quickly opened by hand.

In an exemplary embodiment of the invention, said base hub includes a catheter storage section having a length, wherein said length is less than 80% of a length of a catheter section stored therein.

There is also provided in accordance with an exemplary embodiment of the invention, an extendible catheter comprising:
a base section adapted to remain outside a human body, when the catheter is in use;
an elongate body having a lumen and a distal tip and including a collapsed section stored in said base section; and
a liquid column adapted to apply force to said body adjacent said tip. Optionally, said collapsed section is stored in a folded configuration. Alternatively or additionally, said collapsed section is stored in an axially pleated configuration. Alternatively or additionally, said collapsed section is stored in a coiled configuration. Alternatively or additionally, said collapsed section is stored in an axially folded configuration such that part of said section is inside-out.

In an exemplary embodiment of the invention, the catheter comprises an outer tube out of which said body exits in an uncollapsed state. Alternatively or additionally, the catheter comprises a second collapsed tube inside of said collapsed section. Optionally, said second collapsed tube is a balloon inflation tube.

In an exemplary embodiment of the invention, the catheter comprises a feeding nozzle for uncollapsing said collapsed section.

There is also provided in accordance with an exemplary embodiment of the invention, a catheter with a mechanically activated fluid valve, comprising:
an elongate body having a lumen, said lumen defining a fluid path;
a tool activated by said fluid and situated at a distal section of said elongate body;
a fluid valve at said distal section adapted to selectively convey fluid to said tool; and
a mechanical actuator coupled to said valve and extending outside of said body to control said valve. Optionally, said tool comprises a fluid-inflated balloon. Alternatively or additionally, said catheter is adapted to have a distal section thereof extended distally by said fluid.

In an exemplary embodiment of the invention, said actuator rotates said valve. Alternatively, said actuator retracts a blocking section of said valve. Optionally, said actuator retracts a blocking section of said valve such that in a maximally retracted position the blocking section allows for passage of fluid from said lumen to said tool.

There is also provided in accordance with an exemplary embodiment of the invention, a method of deploying a catheter-carried tool, comprising:
inserting an extendible catheter into a blood vessel of a body; and
extending a distal section of the catheter to reach a target area, by at least a distance of 50 mm. Optionally, the method comprises activating said tool at a distal end of said extended section. Optionally, said extending comprises extending by providing fluid pressure into said catheter.

In an exemplary embodiment of the invention, said tool comprises a balloon.

In an exemplary embodiment of the invention, inserting comprises inserting along a guide wire.

In an exemplary embodiment of the invention, inserting comprises inserting through a guiding catheter/sheath.

In an exemplary embodiment of the invention, the method comprises advancing said catheter after said extending.

There is also provided in accordance with an exemplary embodiment of the invention, a method of testing a catheter, comprising:
attaching the catheter to a source of hydraulic pressure; and
increasing said pressure to extend a distal section of the catheter by at least 50 mm.

### BRIEF DESCRIPTION OF THE FIGURES

Exemplary, non-limiting embodiments of the invention will be described below, with reference to the following figures, in which the same elements are marked with the same or similar reference numbers in different figures:
Fig .1 is a schematic illustration of an exemplary catheter system in use, in accordance with an exemplary embodiment of the invention;
Fig. 2 is a cross-sectional view of a catheter advancing system in accordance with an exemplary embodiment of the invention;
Fig. 3 is a flowchart of a method of using a catheter system in accordance with an exemplary embodiment of the invention;
Figs. 4A and 4B show a catheter system in which an inner tube extends relative to an outer tube, in accordance with an exemplary embodiment of the invention;
Fig. 4C shows a stop mechanism for a catheter, in accordance with an exemplary embodiment of the invention;
Figs. 5A and 5B show a variant of the catheter system of Figs. 4A and 4B, in which a short seal is used, in accordance with an exemplary embodiment of the invention;
Fig. 5C is a close-up of a section of the catheter of Fig. 5A, showing a seal in accordance with an exemplary embodiment of the invention;
Fig. 5D is a close-up of a hub section of the catheter of fig. 5A, in accordance with an exemplary embodiment of the invention;
Fig. 6 shows a catheter system with a guide wire in contact with working fluid, in accordance with an exemplary embodiment of the invention;
Fig. 7 shows a catheter system that is a variant of that of Fig. 6, with a forward section enlarged, in which an extended seal is used, in accordance with an exemplary embodiment of the invention;
Fig. 8A shows a catheter system, in which a single lumen is used for both extension and balloon inflation, in accordance with an exemplary embodiment of the invention.
Figs. 8B and 8C show variants of the catheter of Fig. 8A, showing balloon lumen provision methods, in accordance with exemplary embodiments of the invention;
Fig. 9A shows a catheter system, in which an inner tube thereof is collapsed outside the body, in accordance with an exemplary embodiment of the invention;
Fig. 9B shows a variant of a hub base of the catheter of Fig. 9A, in accordance with an exemplary embodiment of the invention;
Fig. 9C is a top view of a pleated tube, in accordance with an exemplary embodiment of the invention;
Fig. 10 shows a catheter system similar to that of Fig. 9A, except that a separate tube is provided for inflation of a balloon section of the catheter, in accordance with an exemplary embodiment of the invention;
Fig. 11 shows a catheter system in which a single lumen is collapsed by axial folding thereof, in accordance with an exemplary embodiment of the invention;
Fig. 12 shows a back section of a catheter system, in which a separate tube is provided for a balloon section of the catheter, in accordance with an exemplary embodiment of the invention;
Fig. 13 shows a catheter system with a single lumen, in which a mechanically actuated valve is provided, to selectively allow inflation of a balloon section thereof, in accordance with an exemplary embodiment of the invention.
Figs. 14A and 14B show a catheter system, similar to that of Fig. 13, with a different back limiter design, in accordance with an exemplary embodiment of the invention;
Figs. 15A and 15B show a catheter system, similar to that of Fig. 13, with a different valve design, in accordance with an exemplary embodiment of the invention; and
Fig. 16 shows a catheter with an external balloon inflation tube, in accordance with an exemplary embodiment of the invention.

### DETAILED DESCRIPTION OF EXEMPLARY EMBODIMENTS

### Overview

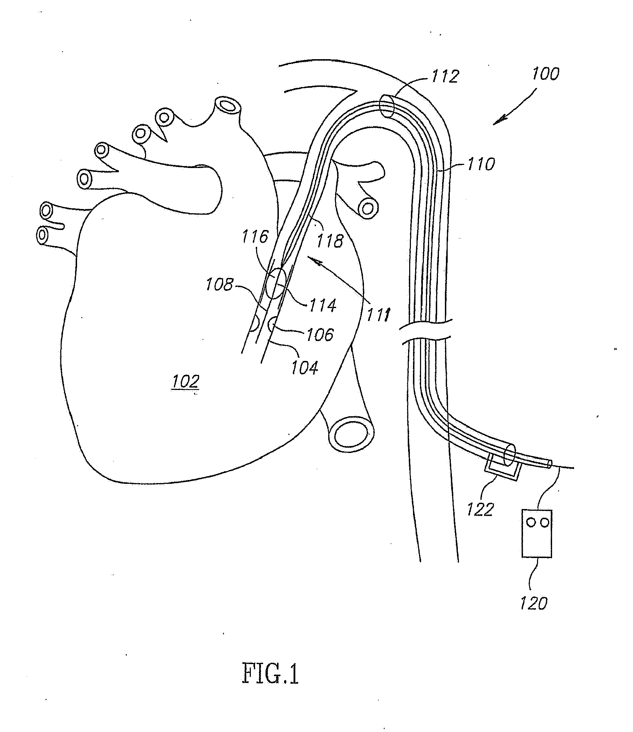

Fig. 1 is a schematic illustration of an exemplary catheter system 100 in use, in accordance with an exemplary embodiment of the invention. A heart 102 includes a coronary vessel 104 with a narrowing 106 (e.g., caused by arteriosclerosis plaque, an old stent, etc. or another types of diseased tissue, such as an emboli). Catheter system 100 includes a guide wire 108 on which rides a catheter 110, at least partly in a guiding catheter/sheath 112. Catheter 110 has a distal tip 111, which optionally includes a balloon 114 and/or a stent 116. In an exemplary embodiment of the invention, a distal section 118 of catheter 110 is advancible from sheath 112, using a force applied at or about distal section 118, as described below. A control system 120 is optional used for controlling this advance. A lock 122 is optionally provided to lock catheter 110 to sheath 112 and/or lock movement of distal section 118 to sheath 112 and/or the rest of catheter 110.

It should be noted that while the figure shows navigation to a coronary vessel, a similar system may be used for navigation to a blood vessel in the brain or another organ. Also, a vascular (or non-vascular) problem other than a narrowing may be treated, for example, radiation delivery to a tumor.

As will be described below, in some embodiments, guiding catheter 112 and/or guide wire 108 are omitted. Alternatively or additionally, the relative placement (e.g., which encloses which) is changed in some embodiments from what is shown in Fig. 1.

### Exemplary catheter with moving outer tube

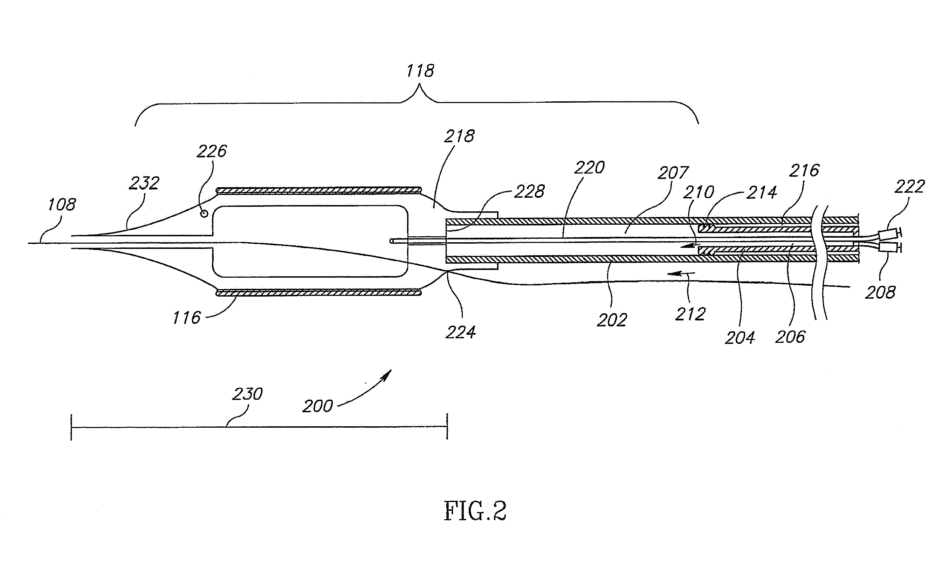

Fig. 2 is a cross-sectional view of a catheter 200, in accordance with an exemplary embodiment of the invention. Catheter 200 has an outer tube 202 and an inner tube 204, with a lumen 206. Outer tube 202 is sealed at a distal end 228 thereof, for example by an optional balloon 218, described in more detail below.

In use, a pressure source 208, for example a syringe, is used to inject fluid into lumen 206. If a liquid is used, this causes pressure increase in a lumen section 207 of outer tube 202 that is contiguous with lumen 206. A sliding fluid seal 214 is provided between tubes 202 and 204. Thus, tube 202 acts as a piston for a cylinder body defined by tube 202. Increase in the internal pressure of lumen 207 causes relative motion of tube 204 (the piston) and tube 202 (the cylinder). In an exemplary embodiment of the invention, tube 202 is freer to move and it moves by advancing balloon 218 in a direction 212, which is also associated with flow of fluid in a direction 210 into lumen section 207. It should be noted that the pressure increase applies force to distal end 228 of tube 202, near the distal end of catheter 200 itself. For example, a distance 230 between a tip 232 of balloon 218 and end 228 can be as small as 30 mm or less, such as 20 or 10 mm or less. Optionally, an inversion balloon is used, in which tip 232 is inverted inwards.

Optionally, seal 214 comprises a gasket, optionally formed out of tube 204, for example as a series of rings. Alternatively, tubes 202 and 204 are a snug fit, (at least for a length of an allowed relative advance) and no special seal is required. If an inter-tube volume 216 exists, it may be provided with a lubricant. Optionally, this lubricant serves as a seal. In an exemplary embodiment of the invention, the lubricant comprises a hydrophilic material as known in the art which swells when contacting water, thereby sealing, and also becoming greasy when contacting water. In an alternative embodiment of the invention, volume 216 serves as a conduit for leaking fluid. Alternatively or additionally seal 214 is a leaky seal, for example, to ensure fluid in volume 216 to serve as a lubricant and/or to serve as a pressure valve which prevents over pressuring of lumen section 207. Tube 202 is optionally substantially non-expandable, for example, expanding less than 1% at 20 atmospheres. Optionally, one or both of tubes 202 and 204 are adapted to not stretch axial under working conditions. In one example, one or more longitudinal fibers (e.g., plastic or metal wires) are embedded in the tubes, to add tensile strength to the tubes. In one example, three or four circumferentially evenly spaced wires are used.

In an alternative embodiment of the invention, no real seal 214 is provided. Instead, the force on end 228 is a function of the rate of fluid inflow and the rate of fluid outflow via volume 216. In general, outflow is inhibited by friction and boundary effects in volume 216. These types of effects in lumen 206 can be overcome by increasing the pressure, thus generating an inequality between inflow and outflow. This rate may be controlled, for example, by increasing or decreasing the pressure provided by source 208. Thus, it is possible to more finely control the force that advances balloon 218, for example to prevent inadvertent damage or to allow passing of sharp bends and/or narrowings.

In an exemplary embodiment of the invention, balloon 218 is provided at the distal end of catheter 200. A separate inflation lumen 220 (e.g., a tube) is optionally provided inside of tubes 202 and 204, and is optionally inflated by a separate pressure source 222. Exemplary variations on methods of inflating balloon 218 and providing fluid thereto, are described below in other embodiments of the catheter. In other embodiments of the invention, a different tool is provided instead of or in addition to a balloon and tube 220 may be used, for example to carry wires and/or materials other than for inflation. In an exemplary embodiment of the invention, tube 220 is a metallic flexible tube, which may assist in reducing kinking and/or enhancing pushability.

Guide wire 108 optionally exits through an exit hole 224, for example after passing through a dedicated tube (not shown) in balloon 218. Other options, for example using well known balloon designs may be used. Some such options and new options are described below in other embodiments.

One or more radio-opaque markers are optionally provided, for example, a marker 226 near a distal end of catheter 200, at seal 214 and/or at end 228. These markers may be used for positioning catheter 200 at various operational stages, for example as described below and/or for estimating a degree of extension of the catheter.

While sheath 112 (Fig. 1) is not shown, it may be provided. Optionally, the sheath will reach until adjacent seal 214. Guide wire 108 optionally travels in the sheath between the sheath wall and outer tube 202 of catheter 200.

Optionally, seal 214 is formed by belling out inner tube 204, for example using heat. In this construction, when fluid pressure increases, it urges the belled section more strongly against outer tube 202, increasing sealing.

### Exemplary process

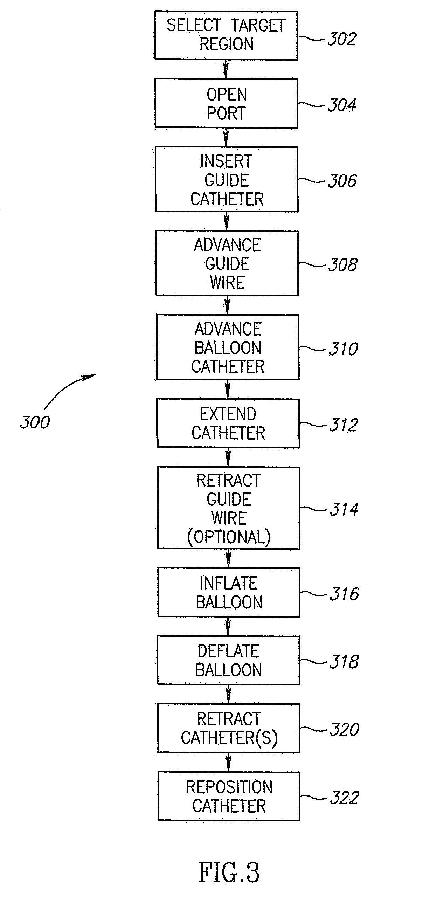

Fig. 3 is a flowchart 300 of an exemplary process of deploying catheter 200, in accordance with an exemplary embodiment of the invention. As will be noted below, the catheters of the invention may be used with other processes as well.

At 302, a decision as to where to guide the catheter, is optionally made. In some cases, for example if lumen 222 is used to inject contrast fluid during a diagnosis procedure, the exact target area may not be known ahead of time. In other cases, a particular narrowing 106 is to be treated by balloon and/or stent.

At 304 a port is opened into a main artery or vein (e.g., a femoral artery), depending on the approach used to reach a target area in the body.

At 306, guiding catheter/sheath 112 is optionally inserted through the port. In the example of a coronary artery, the sheath may be inserted as far as the aorta or even into a main coronary artery.

At 308, guide wire 108 is optionally advanced through sheath 112 and to narrowing 106. Various guide wire navigation techniques as known in the art may be used. In some embodiments of the invention, two guide wires are used. First a thick guide wire and then a thin guide wire, for example as known in the art of catheter navigation.

At 310, catheter 200 is advanced along guide wire 108 (if any) and through sheath 112 (if any) to within a certain distance from narrowing 106. This distance may depend on several factors. For example, a standardized method may be to advance to about the distal end of sheath 112. Alternatively or additionally, catheter 200 may be advanced by pushing until as far as it can be advanced. Alternatively or additionally, catheter 200 may be advanced to within an extension distance ability thereof from narrowing 106.

At 312, source 208 is activated to increase the internal pressure in lumen 207, causing an advancing of outer tube 202 and balloon 218. In an exemplary embodiment of the invention, as the advancing force is applied at near the distal tip of catheter 200, it is more probably that most of the force is directed to advancing the catheter along guide wire 108 rather than to digging a part of catheter 200 into a wall. Optionally, if needed, a pressure burst is applied to lumen 207, to help pass through narrowing 106. As will be noted below, over advancing of the balloon is optionally prevented by restraining tube 202 relative to tube 204.

At 314, guide wire 108 is optionally retracted. In some embodiments, guide wire 108 is removed earlier. In others, guide wire 108 stays in place until the end of the procedure.

At 316, balloon 218 is optionally inflated (e.g., using pressure source 222), optionally deploying stent 116 (if it is provided). Other treatments may be applied instead or in addition to balloon expansion and stent placement In an exemplary embodiment of the invention, lock 122 is applied prior to inflation to prevent motion of balloon 218 during inflation. Optionally, the position of balloon 218 is verified using imaging techniques (e.g., fluoroscopy, CT imaging and/or MRI imaging), prior to inflation.

At 318, balloon 218 is optionally deflated, so that it can be retracted relative to stent 116.

At 320, catheter 200, sheath 112 and/or guide wire 108 are retracted. The retraction may include reversing the pressure at source 208 to cause retraction of tube 202 relative to tube 204. In an alternative embodiment, catheter 200 is simply pulled back.

At 322, catheter 200 is optionally repositioned, for example, by re-advancing tube 202 relative to tube 204.

### Variations on process

There are many techniques of advancing catheters known in the art. Catheter 200 may be used with many of these techniques, optionally with suitable modifications so that the ability of some embodiments of the invention to extend a catheter forward, are utilized.

In one example of an alternative catheter advancing technique, a guide wire and a catheter in accordance with the present invention are advanced as a pair. The guide wire is advanced a short distance and then the catheter is pushed forward and/or extended forward the short distance.

In another example of an alternative catheter advancing technique, catheter 200 is advanced without a guide wire, and is optionally provided with a soft tip.

In another example of an alternative catheter advancing technique, sheath 112 is not provided and catheter 200 is advanced along the guide wire. A potential disadvantage of this technique is that a considerable length of tube 202 is pulled along the blood vessels wall every time catheter 200 is extended forward. The extension mechanism may be used, for example, starting from the entry into the femoral artery or at some intermediate point. Alternatively, catheter 200 is pushed as far as it can be easily pushed before extending catheter 200. If a sheath is provided, for example, extension of the catheter may start at a point other than the entry into the coronary system, for example, before the aortic arch or after one or two branches of coronary arteries.

In another example of an alternative catheter advancing technique, catheter 200 is used to crawl forward, in relatively small steps. In this technique, end 228 is extended and then tube 204 is advanced. Optionally, end 228 is retracted during the advance of tube 204 so that end 228 stays in a same position relative to a blood vessel. Optionally, this technique uses tube 202 is a slightly more rigid guide wire for tube 204.

In another example of an alternative catheter advancing technique, catheter 200 is extended as far as it can be extended and then retracted so that balloon 218 is in the correct position. Alternatively, once the correct position is reached, catheter 200 is pulled back and extended at the same time and/or in small increments so that balloon 218 remains in its correct position. This method is useful for catheter designs (described below) in which a same lumen is used for extending catheter 200 and for expanding balloon 218, and by completing the extension there is no danger of the balloon expansion moving balloon 218. In other embodiments, a position lock is used to prevent such motion.

### Controller logic

Referring back to Fig. 1, controller 120 which is used to control the motion and/or extension of catheter 200 may be of various types and/or abilities. Optionally, controller 120 is manually controlled. Alternatively it is remotely controlled, for example as a robot. In some embodiments, some of the controlling logic is distributed to other parts of the catheter, for example to the moving tubes.

In an exemplary embodiment of the invention, catheter 200 is advanced using a fixed volume technique, in which controller 120 provides a certain increase in the volume of the working fluid, which translates into a certain increase in the volume of lumen 207 and therefore a certain extension of tube 202.

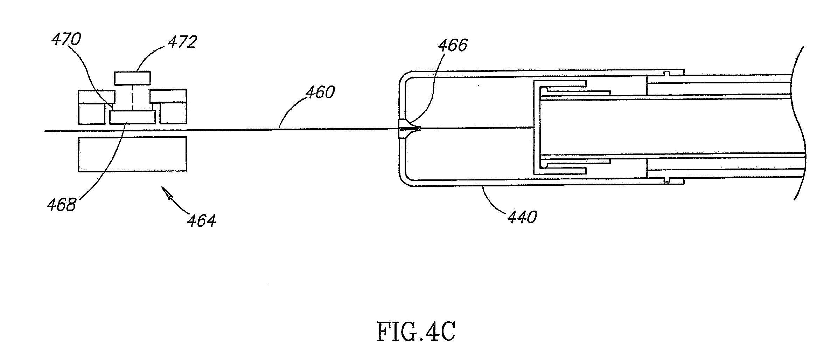

In an alternative embodiment of the invention, a force based technique is used, for example if seal 214 is leaky. In this technique, controller 120 is used to apply a force, for example gradually or as an impulse, to cause an extension of tube 202. Once a desired extension is achieved (or shortly before, to accommodate response time) the force is stopped. Optionally, a combined force/position extension technique is used, in which a maximum advance (or a step size) is set by coupling a stop to each of tubes 202 and 204. Force is then applied to extend or retract tube 202, with the total extension (or retraction) being limited to the distance between the stops. An exemplary such controller is shown in Fig. 4C, below.

It should be noted that, in some embodiments of the invention, the force needed to achieve an advance increases as a function of the number and sharpness of the bends across which the catheter was already extended.

Optionally, a stop is provided, for example as described below, for example a lip on one or both of the tubes, which prevents extension of tube 202 beyond a certain amount (e.g., when the lips meet).

Optionally, controller 120 is used also for retracting of tube 202 towards tube 204, for example by reducing a working fluid volume or by applying a negative pressure.

Various power sources may be used. For example, a push syringe or a syringe with a piston that is screwed into its body may be used. In an alternative manual embodiment, a knob which rolls along a tube is used to increase pressure in the tube by forcing working fluid to advance along the tube. In an alternative embodiment, an electrically controlled pump or fluid or pressure source is used. Optionally, the force is applied non-hydraulically (e.g., using a rigid object, such as a stylet) from the outside the body, which force is conducted to a fluid filled chamber inside the body, to provide the hydraulic extension described herein.

Optionally, controller 120 indicates a degree of extension, for example showing a marking made on tube 202 in a window thereof. Alternatively, the degree of extension may be measured using an optical encoder. Optionally, the amount of extension for a given amount of pressure or force is learned and may be used, for example, while retracting, to retract an amount equal to the last advance. Alternatively or additionally, the extension is tracked for the purpose of providing a closed loop control, in which a user requests a step of extension and pressure is stopped once such an extension is determined. In an exemplary embodiment of the invention, the controller comprises a micro-controller chip with a memory. Optionally, such a memory is used to learn and/or store values for static and dynamic friction of the catheter extension mechanism, for example determined during a calibration process in manufacture or during use.

Controller 120 optionally includes other types of extension sensors, for example an optical encoded which reads markings on the extending tube. In an alternative embodiment, one or more radio-opaque markers are provided on the extending and/or non-extending tubes, so that their relative position is visible using x-ray imaging.

Controller 120 optionally includes other types of sensors, for example, a leakage sensor and a working fluid pressure sensor. Optionally, an accurate pressure gauge, for example a digital gauge is used. Leaks may show as a gradual pressure loss.

In an exemplary embodiment of the invention, controller 120 and/or catheter 200 include limiting means. In one example, a maximum advance per step is limited. Alternatively or additionally, controller 120 can lock two or more of guide wire 108, sheath 112, inner tube 204 and outer tube 202. Which pairs can be locked depend, for example on the particular embodiment. In another example, a maximum pressure limiter is provided, for example a valve may be provided outside the body, to leak over-pressure. The maximum pressure is optionally settable. Alternatively, a drain valve may be defined from lumen 206 to volume 216.

Optionally, controller 120 can also advance or retract the whole of catheter 200, for example by moving inner tube 204. Optionally, controller 120 can synchronize the movement of tube 202 and tube 204, so that the crawling method described above is achieved, e.g., tube 202 is retracted even as tube 204 is advanced. It should be noted that this type of synchronization can also be achieved with a mechanical controller in which rotation of a knob simultaneously advances tube 202 and reduces volume and/or pressure in lumen 206.

While many of the examples describes a liquid working fluid, such as saline solution or other suitable materials may be used, for example bio-compatible materials or lubricating materials. Optionally, an anti-coagulant is provided in the fluid. In embodiments where the fluid is expected to leak to the treated area, various treatment pharmaceuticals may be provided in the working fluid. Optionally, a radio-opaque component is provided in the fluid. In some embodiments of the invention, a gaseous working fluid, such as CO₂, is used. It should be noted that volume 216 can provide a safe path for any such gas to leave the body without coming in contact with any tissue.

### Materials and structure

Catheters in accordance with exemplary embodiments of the invention may be made of various materials, including those known in the art of catheter construction, for example, polyethylene, nylon, PBX, Teflon and other plastics, rubbers and latex. Various coatings are optionally provided, for example, silicone or hydrophilic or hydrophobic coatings. Optionally, a same coating is used on an extending tube both to reduce friction with an enclosing, non-extending tube and to reduce friction or other negative interaction with blood flow and/or blood vessel walls.

In particular embodiments of the invention, the catheter, or at least the extending part thereof is made of a softer material than would be useful for pushing a catheter from outside a body. For example, the catheter or the extending part may be as soft as a softest section (e.g., a most distal section proximal of the balloon) of a standard catheter such as J&J Cordis E95, J&J Cordis "Aqua" (e.g., T3) or Boston Scientific "Maverick". Some elasticity is often useful. In one example, the catheter may be too soft to be effectively and reliably pushed more than 30 cm or 50 cm in the body, in straight sections or in convoluted sections, such as in the coronary system or brain system. In brain applications, the extended part may be longer, softer and of a smaller diameter than in coronary applications.

Exemplary outer diameters of the extending section of the catheter are 2 mm, 1.5, 1 mm, 07 mm, 0.5 mm or smaller, intermediate or larger diameters. The non-extending sections can have an outer diameter of, for example, less than 3 mm or less than 2 mm or a smaller, intermediate or greater diameter.

In some embodiments of the invention a metallic tube is used as an inflation lumen for the balloon, also optionally providing some kink resistance, pushability and/or ability to apply tapping and/or vibration to obstructions (e.g., using a vibrating means as known in the art).

A non-moving tube (e.g., an outer tube), if it stays in the aorta (or other main vessels) may be stiffer than an extendible tube (e.g., an inner tube). Optionally, such a stiffer tube may be stiff enough to act as a guiding catheter and allow a separate guiding catheter to be omitted. In such an embodiment, the guiding catheter and balloon carrying section are optionally advanced together over the guide wire, until the point where only the balloon carrying section is advanced.

In an exemplary embodiment of the invention, the catheter can be made with smaller dimensions, for example having an outer diameter of between 0.4 and 3 mm, or smaller.

While the term "tube" is used, various, non-circular cross-sections may be used as well. In addition, while concentric tubes are generally described, this is not a required feature and the tubes may be non-concentric.

In an exemplary embodiment of the invention, considerable forces can be applied at the tip of the catheter. For example, a 5 French catheter may apply as much as 160 grams or more at its distal tip. Optionally, smaller forces, such as up to 140 or 100 grams are applied. Alternatively, greater forces, such as 200 grams or more are applied. These forces may reduce for smaller diameter catheters, for example being a linear or quadric function of the catheter dimensions. Optionally, the force applied is a significant fraction (e.g., 20%, 30%, 50% or more) of a product of the catheter cross-section and the applied pressure. As can be appreciated, this may depend, for example on the hydraulic cross-section of the catheter as well as on the pressure loses along the catheter. Optionally, the achieved forces have a high raise rate.

### Catheters with moving inner tube

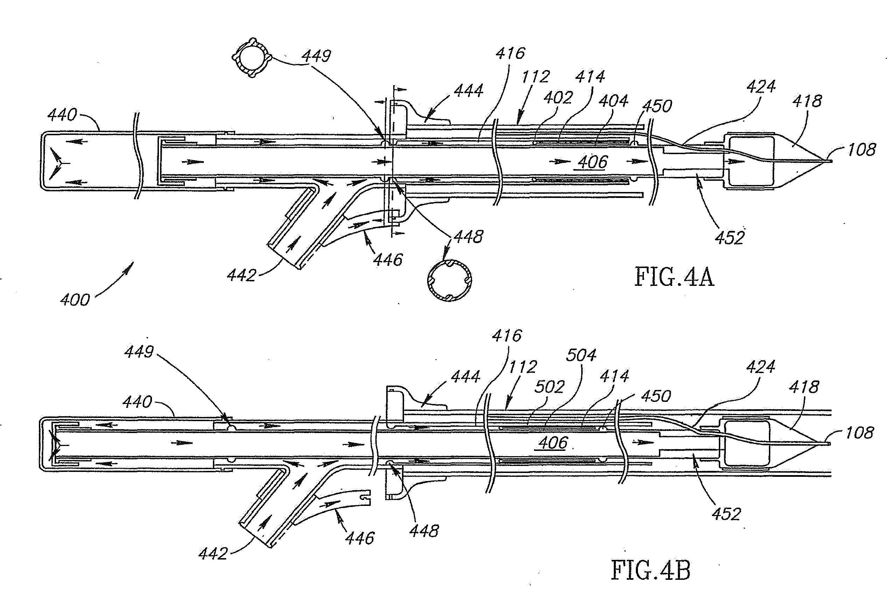

Figs. 4A and 4B show a catheter 400 in which an inner tube 404 extends relative to an outer tube 402. A balloon 418 is provided at a distal end of inner tube 404. A lumen 406 of inner tube 404 serves for inflation of balloon 418, while a volume 416 between the two tubes serves to provide a fluid column for advancing tube 404 and balloon 418. One possible advantage of providing a working fluid in volume 416 is to reduce friction between tubes 402 and 404.

While a seal between the two tubes can be axially short (e.g., as described below), in an exemplary embodiment of the invention, a relatively long sealing tube 414 is used. Tube 414 can be, for example, 10 mm, 20 mm, 40 mm, 80 mm, 200 mm or longer. One potential advantage of using a long tube is that the contact with the inner and/or outer tube can be less tight while still inhibiting or preventing leakage (e.g., based on boundary effects).

Optionally, sealing tube 414 has more than one function. One optional function, sealing, may be provided by a part of the tube (e.g., even a very short section thereof, as in other seals described herein). Another optional function is prevention/reduction of kinking. Optionally, sealing tube 414 is made slightly rigid. Another optional function, force extension, is described next. In other embodiments, for example as described below, the working fluid is inside inner tube 404, so it is less likely to leak out. Optionally, the working fluid includes a contrast material, so that leakage can be identified using imaging, for example fluoroscopy.

Optionally, the distal end of tube 414 contacts one or more protrusions 450 formed on inner tube 404. As the fluid column abuts the proximal part of seal 414, seal 414 conveys the applied force to protrusions 450 and thereby advances tube 404. This optionally allows the force to be applied at a more distal location. Optionally, at least part of seal 414 can extend past outer tube 402. Fig. 4A shows catheter 400 in an extended configuration and Fig. 4B in a non-extended configuration. Optionally, seal 414 is mounted to one of tubes 402 and 404.

In an exemplary embodiment of the invention, catheter 400 has a maximum extension length, for example 300 mm for coronary uses, 500 mm for brain uses or 50 mm for advancing past obstructions. Optionally, this maximum extension length is enforced by at least one pair of stops, a stop 448 on outer tube 402 and a stop 449 on inner tube 404. It should be noted that these stops, even if not at a proximal end of tube 404, are generally near the most distal end of tube 404, when extended. If multiple stops are provided, fluid pressure may apply a pushing force at multiple points along tube 404. In the embodiment shown, the stops when they contact each other do not prevent further flow of liquid. One potential advantage of this design is allowing retraction by application of vacuum. Another potential advantage is that this allows maximum force to be applied even near an end of the extension. In an alternative embodiment the stops also seal. Optionally the lack of blocking allows the stops to be larger without a danger of inadvertently blocking volume 416. Alternatively or additionally, the stops serve as spacers between the tubes. Multiple spacers, even without a stopping function may be provided for spacing the tubes. Optionally, this reduces friction, to those points of contact. Optionally, the most proximal stop of tube 404 is made by belling an end (or middle section) of tube 404, for example by heat and/or distortion, to have a greater diameter.

In an exemplary embodiment of the invention, multiple axially spaced stops are provided so that the relative extension of the tubes can stop at multiple locations. A small amount of overshoot may be amended, for example by pulling back the catheter or the extended tube. Optionally, the intermediate stops are not absolute, for example a sufficient, optionally known, force will over come them. Alternatively or additionally, relative rotation of the tubes will allow one stop to slide by the other stop. Alternatively or additionally, the stops are located in a base section 440 of the catheter, where a suitable mechanism can be used to expand the outer tube or compress the inner tube so the stops slide by each other. In an alternative embodiment of the invention, stop 448 is provided as part of base 440, and may be, for example, adjusted axially. An exemplary stop design is shown in Fig. 4C, below.

A potential advantage of multiple stops is allowing a user to vary the extension by known amounts, for example extending or retracting to a certain stop or a certain number of stops. For example, after extending to just before a stop, catheter 400 itself may be advanced the distance between stops tube 404 retracted to just after a stop, thereby leaving balloon 418 at a same body location. In another example, a stop can be used to prevent an overshoot effect when applying a high pressure to pass a narrowing or other obstruction, by the extension stopping at the next stop.

A fluid port 442 is optionally provided for injecting fluid under pressure (e.g., using controller 120 or a syringe, as described above). The same port is optionally to inflate balloon 418 by selectively connecting port 442 to inner tube 404. In an alternative embodiment of the invention, once balloon 418 is positioned base 440 is opened and a second or same pressure source is attached to a proximal side of tube 404. Optionally, tubes 402 and 404 are locked prior to such opening. Alternatively or additionally, stops 448 and 449 may be designed to interlock and prevent retraction of tube 404 once advanced. Base section 840 maybe of various lengths. For example, if tube 404 is not folded, it may be, for example, 20, 30 or 40 cm long. If a folded tube is used (e.g., as described below), a shorter section may be provided, for example less than 20 cm.

Optionally, sheath 112 is provided, as described above. Optionally, a sheath lock 446 is provided to lock outer tube 402 to a sheath base 444, to prevent relative motion between sheath 112 and catheter 400. In some embodiments, if such a lock is not provided, rather than tube 404 extending, tube 404 may jump or move away from the body. The locking to sheath base 444 optionally prevents this.

Various mechanism as known in the art may be used for sealing between outer tube 402 and sheath 112, for example, a tight fit, a rubber gasket and a valve.

In the embodiment shown, balloon 418 is provided with a thick base 452, through which guide wire 108 passes and exits through a guide wire port 224 defined in the base. This allows for rapid/exchange type guide wire usage. The distance between port 224 and the proximal end of balloon 418 can vary between embodiments, for example being less than 100 or 50 mm, possibly as little as 10 mm or down to about 0 mm. The distance may also be greater than 0 mm, for example, greater than 5 or 10 mm. In other embodiments of the invention, guide wire 108 passes through the inflation lumen of balloon 218.

In the embodiment shown, guide wire 108 lies outside of sheath 112. In other embodiments described below this can change, for example, the guide wire may be inside or outside the sheath, inside or outside the catheter, in contact with the working fluid and/or inflation fluid or not, passing through sheath base 444 and/or catheter base 440, or not.

When extending catheter 400 past a blockage there may be a danger of overshoot, in which a high pressure applied to overcome the blockage will, once the blockage is passed, translate into a large rapid extension. In an exemplary embodiment of the invention, friction is applied to tube 404, inside base 440, to reduce such overshoot. Optionally, such friction is controllable. Alternatively or additionally, a temporary stop may be place to limit a maximum extension.

A volume-based extension mechanism is optionally used, in which each quantity of fluid injected into port 442 is translated into a pre-determined extension, so overshoot is not a problem. This is optionally provided if catheter 400 has reduced leakage. Optionally, an elastic membrane or chamber is provided in base 440, to allow the injected fluid to translate into a pressure buildup even without extension. Optionally, a pressure relief valve is provided in base 404 to prevent over pressuring (which may cause leakage). Optionally, the release pressure of this valve is user settable. Alternatively or additionally, a valve is formed in inner tube 402, such that fluid can leak from tube 44 into tube 404 if the pressure is too great. Optionally, the elasticity of catheter 400 itself is used to allow for pressure buildup.

### Wire stop

Fig. 4C shows a wire stop mechanism, in accordance with an exemplary embodiment of the invention. A wire 460 is attached to a back section of inner tube 404 and extends out of hub base 440, through a port 4666, for example. When tube 404 extends, it pulls wire 460 along with it. An optional brake section 464 is provided on wire 460 to control such extensions. In one example, a screw 472 attached to a spring 470 and a pad 468 cooperate to allow a friction between pad 468 and wire 460 to be set. At a maximal setting, the distance between brake 464 and port 466 set a maximum extension possible. Optionally, one or more small brakes (not shown), for example bumps in the cable are used to preferentially stop wire 466 when such bumps reach port 466. In another example, brake 464 is an acceleration break which prevents too fast a motion of wire 460 through it. Many acceleration brakes are known in the art, for example utilizing a non-straight bore in brake 464, for wire 460.

In an exemplary embodiment of the invention, valve 466 has the design shown, in which increased intra-hub pressure will cause the port to more snugly engage wire 460 and possibly reduce leakage.

Such a wire stop may be used in other embodiments of the invention. However, it may be useful to attach the wire to a more distal part of tube 404, possibly to a base of balloon 418 (or its equivalent) in some embodiments.

### Short seal variant

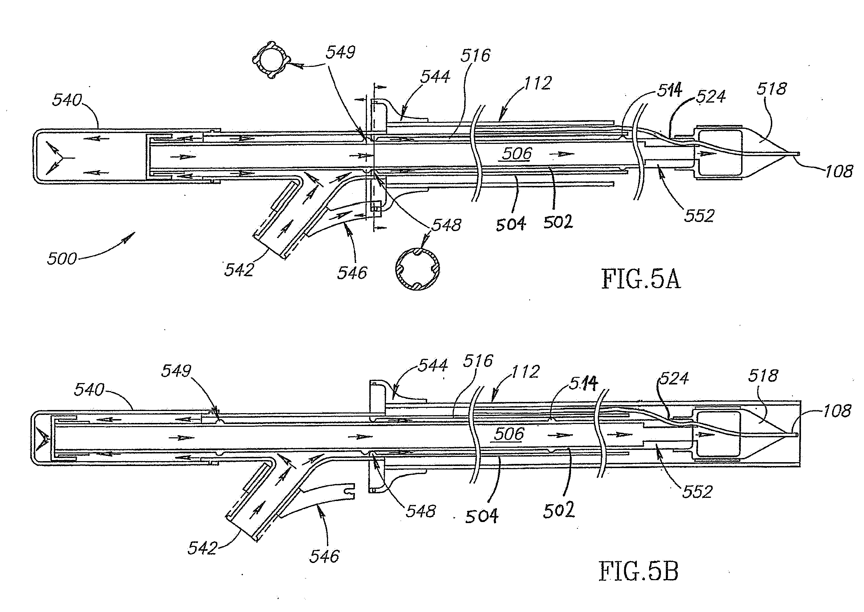

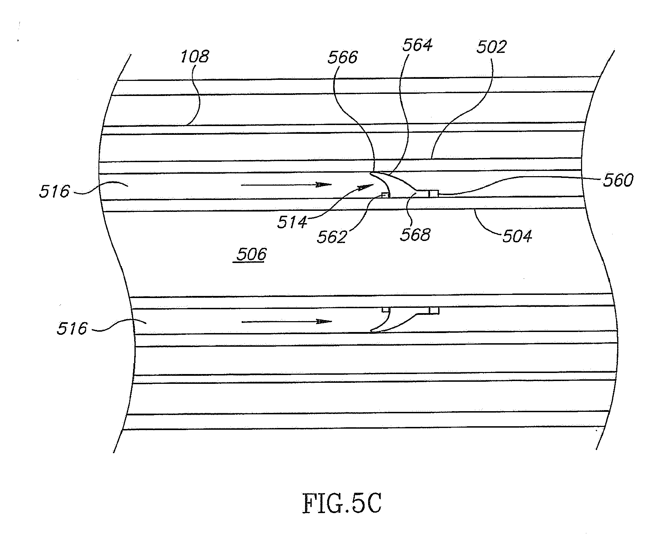

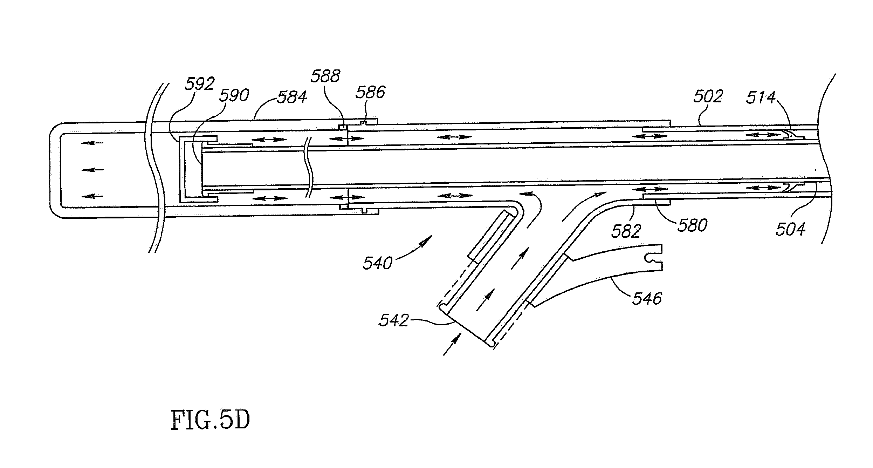

Figs. 5A and 5B show a catheter 500 which is the same as catheter 400, except that sealing tube 414 is replaced by a short seal 514 mounted on an inner tube 504 or an outer tube 502 of catheter 500. The reference numbers in Fig. 5 are the same as in Fig. 4, except for being increased by 100. In this and other figures, same parts with same functions have a the same last two digits, such elements will not generally be re-described.

It should be noted that using a short seal moots the use of tube 414 for advancing force in a distal direction.

Fig. 5C shows the details of an exemplary seal design for seal 514. In this design, seal 514 comprises a base section 568 attached to inner tube 504 and an elongate, more flexible portion 564. Optionally, portion 564 is flexible enough to change its effective diameter, possibly fitting with various outer tube diameters 402. Optionally, this type of seal is used to seal a balloon catheter to a guiding catheter, using the guiding catheter as the outer tube.

In an exemplary embodiment of the invention, seal 514 is mounted between a forward protrusion or adhesive point 560 and a rear protrusion and/or adhesive point 562. There may also be a layer of adhesive (or heat sealing maybe used) to assist attachment.

In an exemplary embodiment of the invention, seal 514 contacts outer tube 502 at a limited tip area 566 of seal 514. Possibly, this reduces friction.

In the embodiment shown, seal 514 has a design which increases the contact force between tip 566 and tube 502, as the fluid pressure inside volume 516 increases. Optionally, a same type of seal, in an opposite direction, is used to prevent ingress of blood into the catheter system.

Other seal designs may be used as well, for example, ridges on one or both tubes, o-rings, short sections of tube and/or magnetic fluids.

### Exemplary hub design

Fig. 5D shows an exemplary hub design, in accordance with an exemplary embodiment of the invention. Hub 540 comprises a body 582 and a back 584. In an exemplary embodiment of the invention, body 582 is attached using an adhesive layer 580 to outer tube 502. Back 584 is optionally transparent so that the extension of tube 504 therein can be viewed

Back 584 is optionally attached to body 582 using a quick connection, for example a snap connection 586, or a half-tum and lock connection. An O-ring 588 optionally helps seal the connection.

After tube 502 is advanced, back 584 is removed and tube 504 is inflated to inflate balloon 518. In an exemplary embodiment of the invention, a cap 592 is provided on a proximal section 590 of tube 504. Optionally, this cap is a screw cap. Alternatively, it is a snap cap. It is noted that during operation of some embodiments of the invention, the pressure in hub base 540 should generally be greater than that of tube 504, ensuring that cap 592 does not fall off. In this design, fluid form port 542 can surround inner tube 504 from all sides. It should also be noted that there is generally sufficient space around cap 592 to allow fluid flow, so that all of base 540 is at a same pressure.

### Variant with second seal and guide wire in liquid

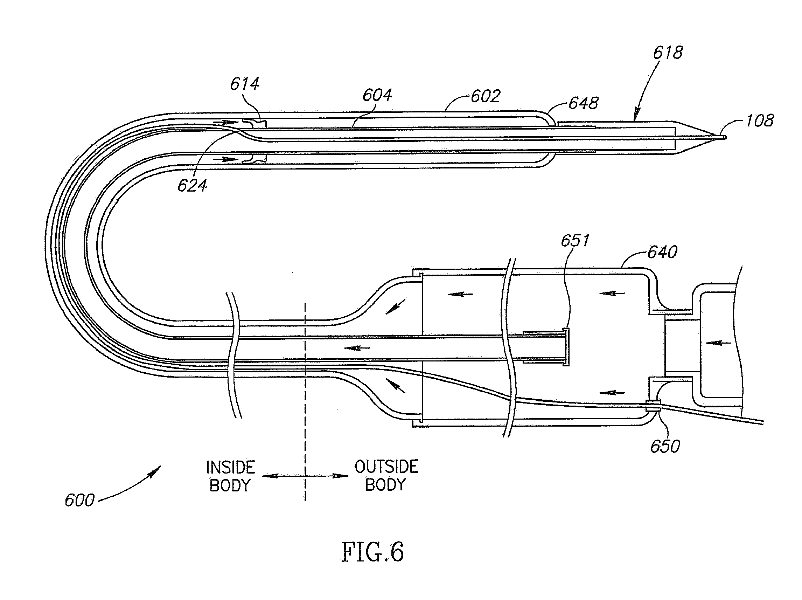

Fig. 6 shows a catheter 600 with several features, any one of which can be used on its own. A first feature shown in Fig. 6 is that outer tube 602 includes a stop 648 at a distal end thereof and which engages a seal 614. Seal 614 also acts as an inner tube stop (e.g., like 449 in Fig. 4). Optionally, this allows the use of a standard balloon catheter to act as an inner tube 604, only requiring attachment of seal 614 thereto. Optionally, stop 648 serves as a second seal to reduce leakage or as a backup.

A potential disadvantage of this design is that seal 614 and the point of application of force to the distal part of catheter 600 are displaced from a balloon tip 618 by the amount of maximum extension.

Another feature shown in Fig. 6 is that a guide wire port 624 of balloon 618 is proximal of seal 614. Thus, guide wire 108 travels in a volume 616 between the two tubes and optionally exits through an exit port 650 in a base 640 of the catheter. Optionally, a special tube is provided for carrying the guide wire to prevent its contact with the working fluid. Alternatively or additionally, a narrow lumen (not shown) is provided for guide wire 108 in tube 604, the lumen being narrow enough that very little or no fluid leaks through.

In an alternative embodiment of the invention, port 624 is distal of seal 614 and an aperture (not shown) is provided for passage of guide wire 108 through seal 614. Alternatively, a port near balloon 618 may be provided, as described above. A removable cap 651 for inflation of tube 604, is shown. Optionally, tube 604 is partially filled with fluid, even prior to balloon inflation, so that tube 604 does not collapse. Alternatively, such collapsing is useful in that it prevents blockage of the volume between the tubes by kinking and bending of the catheters as they are inserted.

### Variant with force applied near catheter tip

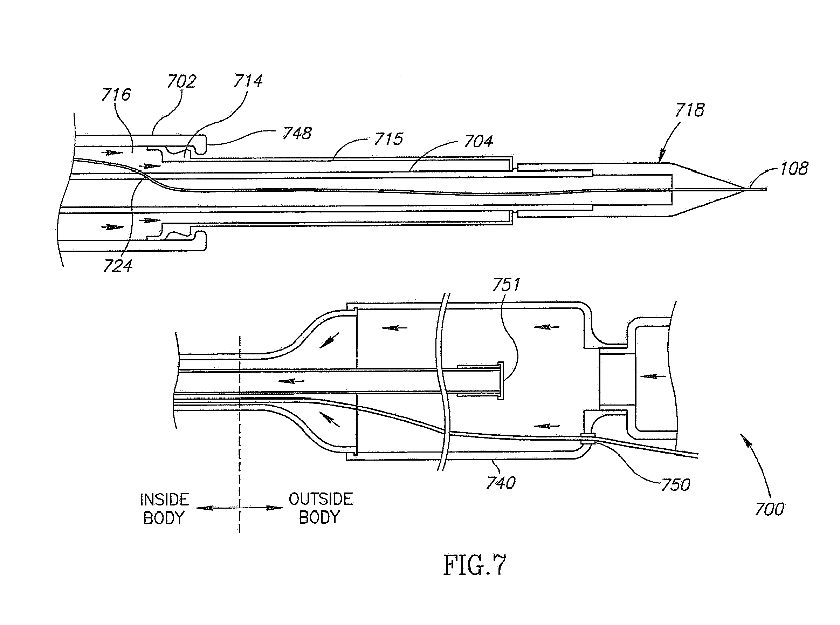

Fig. 7 shows a catheter system similar to that of Fig. 6, in which a stop 748 of an outer tube 702 does not touch an inner tube 704. Instead, a seal 714 includes an extension 715 which has a lumen contiguous with a volume 716 between tube 704 and tube 702. Extension 715 is adapted to slide past stop 748. In this way, the liquid column reaches substantially to balloon 718 (or less, if desired). Optionally, as shown, stop 748 is adapted to engage and stop a proximal portion of seal 714.

A potential advantage of this design is that while the seal between tube 702 and tube 704 is formed of two contacting surfaces (stop 748 and seal 714), this contact is optionally not provided during most of the extension process, possibly reducing friction. Some amount of sealing is optionally provided by seal 714 even without contacting stop 748. Alternatively, stop 748 is in contact with and optionally seals to extension 715. Optionally, as shown, the distal section of seal 714 includes a ratchet mechanism to prevent retraction of tube 704, once completely extended. Alternatively, the design shown is used to offset seal 714 from an end of tube 702. Alternatively or additionally, an offsetting protrusion (e.g., proximal of stop 748, for example by 5 mm) is provided on the inside of tube 702.

In an alternative embodiment of the invention, guide wire 108 passes through a port in seal 714 itself.

### Single fluid lumen catheter

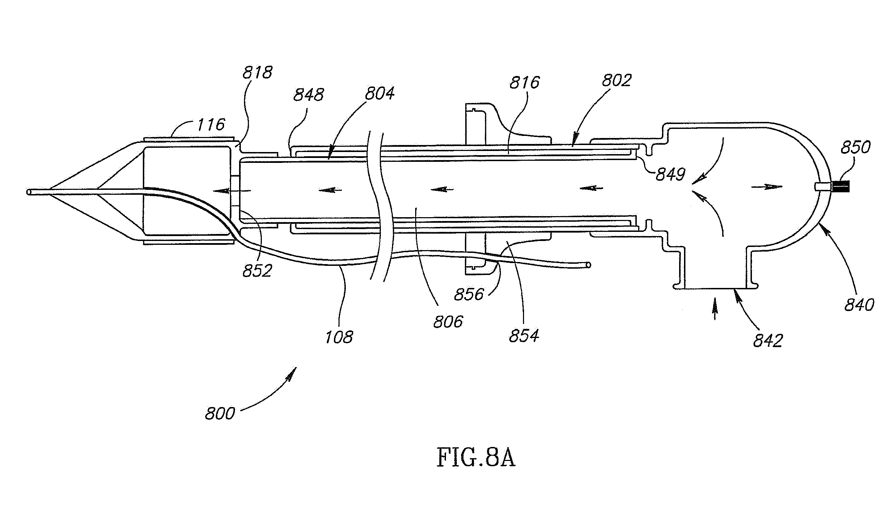

Fig. 8A shows a catheter system 800, in which a single lumen is used for both extension and balloon inflation, in accordance with an exemplary embodiment of the invention. Catheter 800 comprises an outer tube 802 and an inner tube 804 with a lumen 806. Unlike catheter 400, the working fluid for extending catheter 800 is in lumen 806. When liquid is injected through a fluid port 842 in a base section 840 of catheter 800, inner tube 804 and its attached balloon 818 extend. In the embodiment shown, lumen 806 continues unobstructed to the distal tip of balloon 818, where it applies its advancing force. A volume 816 between the tubes is optionally provided with a lubricant. Alternatively or additionally, one or both of the tubes is coated with a low friction coating, for example, a silicone coating or a hydrophilic coating, for example "Rotaglide" by Boston Scientific, USA. Alternatively or additionally, some of the working liquid leaks through an inner tube stop 849 into volume 816. An outer tube stop 848 optionally serves as a seal to block such leakage into the blood stream.

Optionally, the stops between tubes 802 and 804 serve to keep working fluid out of the blood and/or blood out of the working fluid. Optionally, the seal nearest the base of the catheter seals in working fluid (e.g., and using the design of seal 514, faces inwards) and the seal nearest the tip of the catheter seals out blood (e.g., and in the design of seal 514, faces outwards).

In an exemplary embodiment of the invention, balloon 818 is inflated by using a balloon in which a minimum inflation pressure is higher than that used for extension. In one example, a pressure of less than 4 or 2 bar is used for extension and a pressure of at least 10 or 15 bar is used for inflation of balloon 818. In other embodiments, for example if there are many curves, the pressures may increase, for example, up to 10 or 15 atmospheres for the advancing and 15-20 or more for the balloon. In other embodiments, the advancing is easier, and, for example, pressures of under 2 atmospheres serve for advancing, while pressures of 4 atmospheres and above serve for balloon inflation. Optionally, a pressure relief valve 850 is provided in base 840, so that high, inflation-suitable, pressures are not achieved in the working fluid. Once balloon 818 is to be inflated, the valve may be adjusted, deactivated or removed, for example. Alternatively or additionally, an optional pressure valve 852 is provided between lumen 806 and balloon 818 and is opened only by high pressures. Alternative, as described below, a manually actuated valve is used.

Optionally, for example as described below, a push or vibrating wire is inserted through lumen 806, for example to help balloon 818 advance past obstructions. This is optionally done via the opening of valve 850.

In an exemplary embodiment of the invention, when balloon 818 is to be inflated, tubes 802 and 804 are locked relative to each other. In one embodiment, tube 804 is simply advanced to its end, and then (or at the same time) the whole catheter retracted if needed so balloon 818 is in a correct location relative to narrowing 106. Optionally, a pull wire such as described in Fig. 4C is used for such locking.

In the embodiment shown, guide wire 108 exits balloon 818 through a guide wire port 22 in the base of balloon 818, and guide wire 108 travels outside of catheter 800. In the embodiment shown, an external holder 854 is provided, with a path 856 for arranging guide wire 108.

### Coiled storage

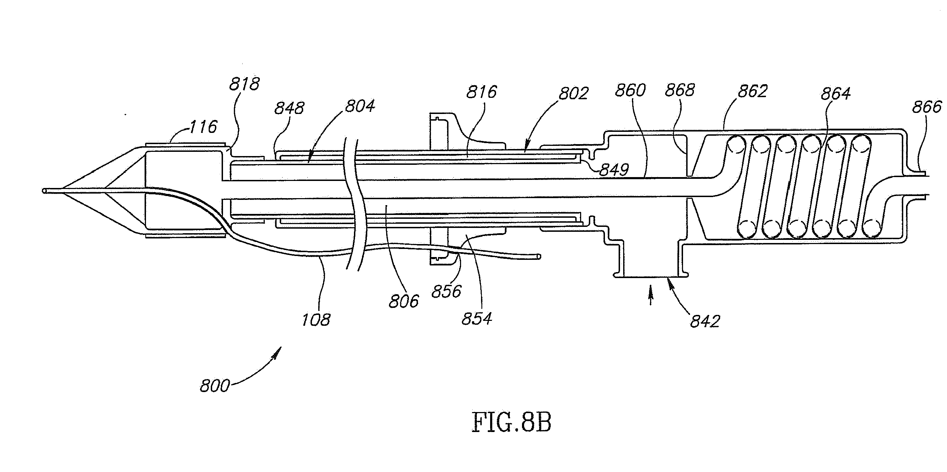

Fig. 8B shows a variant of Fig. 8A, in which a separate balloon inflation tube 860 is provided. This tube is optionally coiled up in a storage section 862. A cone-shaped guide opening 868 is optionally provided to help a coiled section 864 of tube 860 straighten out as pulled by tube 804. A proximal side of tube 860 is optionally attached to a balloon inflation port 866.

Optionally, tube 860 is kept evacuated, so as to minimally interfere with catheter extension and/or to prevent pressure on tube 860 from inflating balloon 818. Alternatively, some amount of fluid is provided in tube 860, for example to assist it in leaving storage section 862 or to prevent kinking or piling up thereof. It should be noted that in some embodiments of the invention, for example in Fig. 4, it may be desirable to provide some fluid into the inner tube (402) to ensure the seal between the inner and outer tubes, which might be compromised by the inner tube collapsing.

### External storage

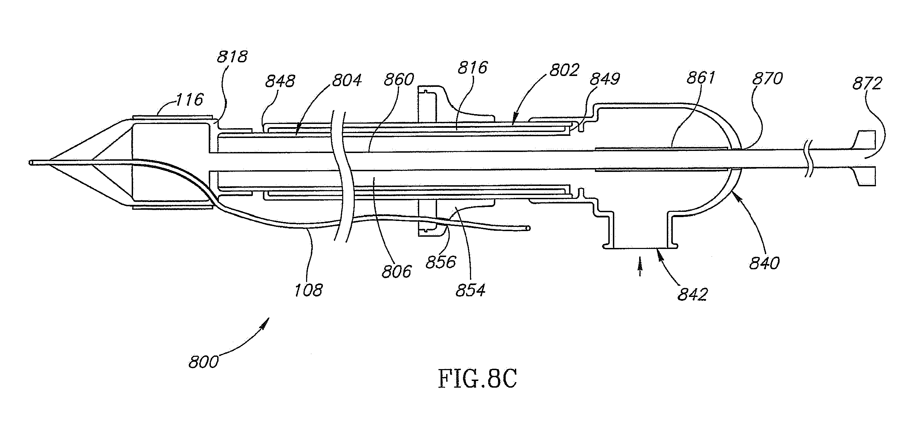

Fig. 8C shows a variant in which tube 860 is stored outside the catheter, for example exiting base 840 via an opening 870 with an external fluid port 872. In such a case, tube 860 optionally serves as a stopping mechanism such as described in Fig. 4C. In this and in other embodiments, a tube section is optionally used to assist sealing. For example, a tube 861 may enclose tube 860 inside base 840 and help seal port 870 from the working fluid and/or to prevent tube 860 from being crushed by port 870. The fit between tubes 861 and 860 is optionally relatively close, to provide a sealing function. Optionally, a suitable sealant or coating is provided to enhance the seal.

Optionally, a tube such as tube 861 is used to seal between a catheter hub and an outer tube, in this or other embodiments.

### Collapsed lumen

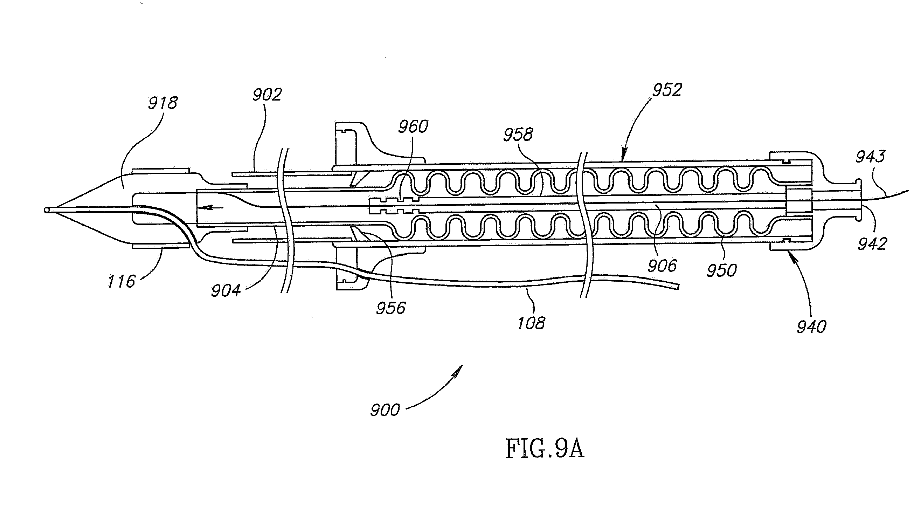

Fig. 9A shows a catheter system 900, in which an inner tube 904 thereof is collapsed outside the human body, in accordance with an exemplary embodiment of the invention. A single lumen 906 of tube 904 can be used, for example as described in Fig. 8A. Collapsing outside the human body allows a larger diameter storage section to be used than might be possible or desirable inside the body. It is noted, however, that in some embodiments of the invention a large diameter vessel may be treated as being outside the body for the purpose of providing a storage area for a small-diameter catheter.

In the embodiment shown, a section 950 of tube 904 is axially collapsed, to have a form similar to that of a folded accordion. Section 950 is optionally stored outside the body, for example, in a storage tube 952. Optionally, an outer tube 902 is provided, for example having a length of 1 meter, within which lies a corresponding section 954 of inner tube 904, in uncollapsed condition. A feeding nozzle 956 optionally feeds collapsed section 950 into tube 902 or into a sheath (not shown).

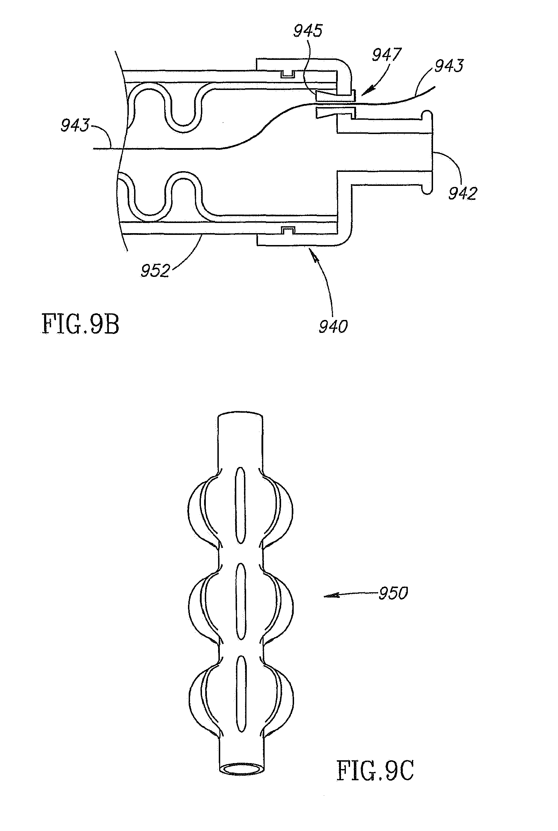

Fig. 9C shows an exemplary design of section 950, in which when the accordion shape is axially extended, pleats (such as found in balloons) optionally form along pre-defined bending lines. These pleats are folded around section 950 as it is pulled through nozzle 956. Optionally, rings of a material with tensile strength are provided in section 850 (and/or in other accordion tubes described below), to prevent its expanding at undesired points. Section 950 is optionally created by inflation of tube 904 into a form with multiple axially separated expansion areas.

Referring back to Fig. 9A, an inner tube or wire 958 is optionally provided to help nozzle 956 form and/or fold such pleats. Optionally, one or more holes 960 in tube 958 provide fluid which pushes the pleats outwards.

In use, fluid pressure is increased via a fluid port 942 of a base section 940 of catheter 900. The increased fluid volume pushes at a balloon section 918 of catheter 900, which pulls some of section 950 out of nozzle 956 and forward.

Optionally, a wire 943 is provided to selectively stop the advance of balloon 918 and/or allow its inflation without advancing. Optionally, if wire 943 is not attached along an axis of catheter 900, the combined effect of applying pressure and preventing advance of wire 943 will cause turning of the tip of balloon 943, which may be used for navigation. A similar mechanism may be used in other embodiments of the invention as well.

Fig. 9B shows a design of an exemplary self-sealing valve port 947, in which increased pressure (e.g., balloon inflation pressure) causes a locking of wire 943. In the design shown, a cone shaped section 945 of port 947 is urged into port 947 (as formed in base 940). As pressure increases it is more strongly urged. The cone shape causes increased pressure on wire 943, and this increased friction, as the cone is pushed in more. Optionally, port 947 is made fast responding. Alternatively, it may be slow responding. Once activated, port 947 is optionally released by being manually pushed in.

### Double collapsed lumen

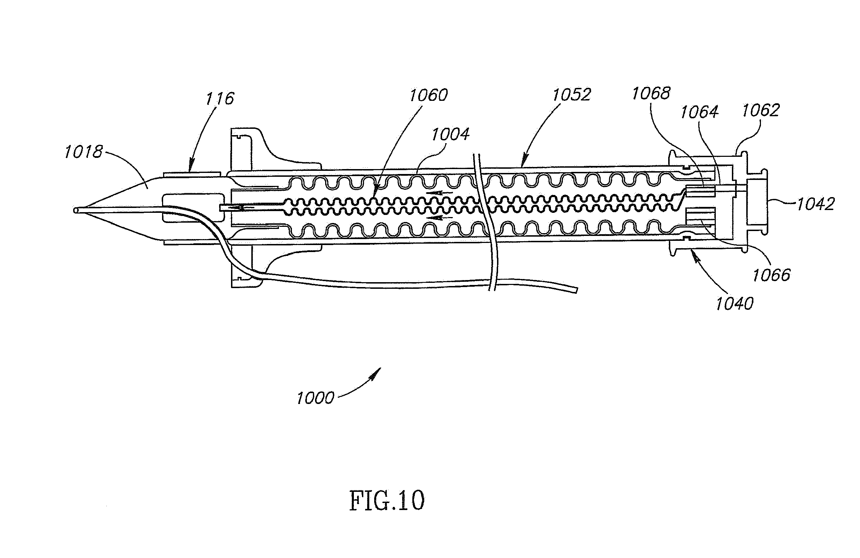

Fig. 10 shows a catheter system 1000 similar to that of Fig. 9A, except that a separate tube 1060 is provided for inflation of a balloon section 1018 of catheter 1000, in accordance with an exemplary embodiment of the invention. In the embodiment shown, tube 1060 is also folded accordion-like. However, it may be stored in a different manner, for example being coiled or folded or extending out of a base 1040 of catheter 1000. A feeding nozzle as in Fig. 9A is optionally provided.

In an exemplary embodiment of the invention, base 1040 includes two ports, one for inflating balloon 1018 and one for extending an inner tube 1004 thereof. Alternatively, as shown, a single port 1042 is used which can be selectively attached to tube 1060 or tube 1004. In the exemplary method shown, port 1042 is mounted on a rotating section 1062 and includes an off-center axial lumen 1064. Base 1040 includes two off-center lumens at different angular positions, a lumen 1066 attached to tube 1002 and a lumen 1068 attached to tube 1060. Rotation of section 1062 selectively aligns its lumen 1064 with one or the other of lumens 1066 and 1068, thus selecting the effect of increased fluid pressure.

### Axially folded lumen

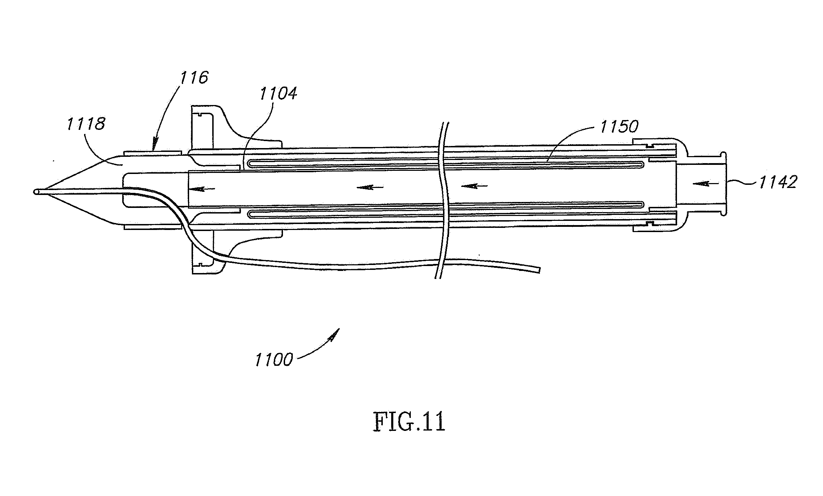

Fig. 11 shows a catheter system 1100 in which a single lumen is collapsed by axial folding thereof, in accordance with an exemplary embodiment of the invention. A folded section 1150 of an inner tube 1104 is stored in a storage tube (or other geometry) 1152 outside the body. When fluid pressure is provided through a fluid port 1142, a balloon section 1118 of catheter 1100 is pushed forward, pulling behind it tube 1104. This causes part of section 1150 to unfold and be fed distally. As in Fig. 9A, an outer tube and/or a feeding nozzle are optionally provided. Optionally, at least section 1150 is tapered, to assist folding.

### Collapsed balloon tube

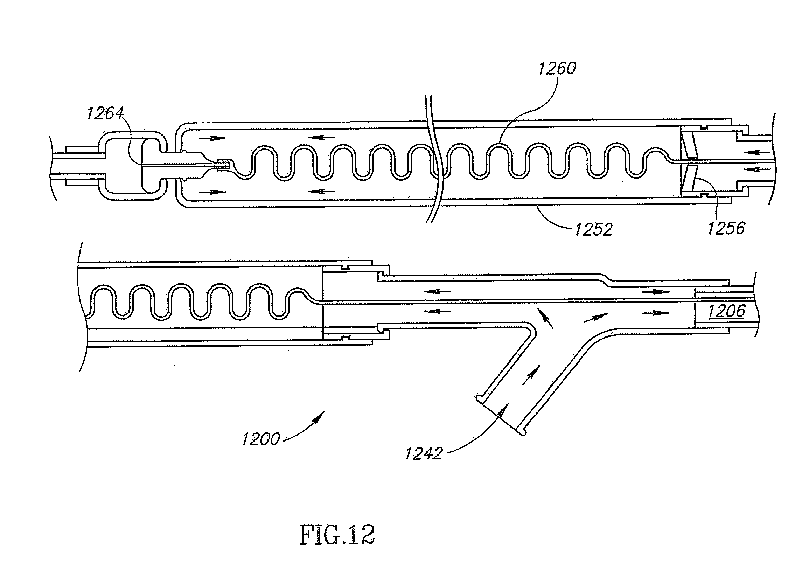

Fig. 12 shows a back section of a catheter system 1200, in which a separate tube 1260 is provided for a balloon section 1218 (not shown) of catheter 1200, in accordance with an exemplary embodiment of the invention.

This back section may be used with many of the catheter designs described herein, in which a separate tube is provided for inflation of balloon 1218, for example, for Figs. 6-8, 10 and 11. In the case of catheter system 1000, for example, a balloon tube 1260 may be folded up in a storage section 1252 which is proximal of storage tube 1052. A feeding nozzle 1256 is optionally provided for feeding tube 1260. A similar design (or other storage designs described herein) may be used for the inner tube in those or other embodiments. Also it is noted that a separate balloon inflation tube may be added in various embodiments, even where none is shown in the figure.

While tube 1260 is shown as having a diameter significantly smaller than that of a lumen 1206, it may be of similar diameter or even act as an inner tube (e.g., as tube 604 of catheter 600 in Fig. 6 or in Fig. 4). While tube 1260 is shown as folded in a pleat fold, it may be arranged otherwise, for example, coiled or folded in a spiral manner (e.g., Figs. 8B and 8C). In use, fluid pressure is provided through a port 1242 and when this advances balloon section 1218, it pulls tube 1260 along with it and out of storage section 1252. Optionally, a separate fluid port 1264 is provided for tube 1260.

Optionally, storage section 1252 is flexible so that pressure on it (e.g., adjacent nozzle 1256) can stop feeding of tube 1260 and possibly halt the extension of the catheter.

Alternatively or additionally, storage section 1252 is squeezable or is attached to a squeeze bottle, for increasing working fluid pressure.

### Mechanical force application

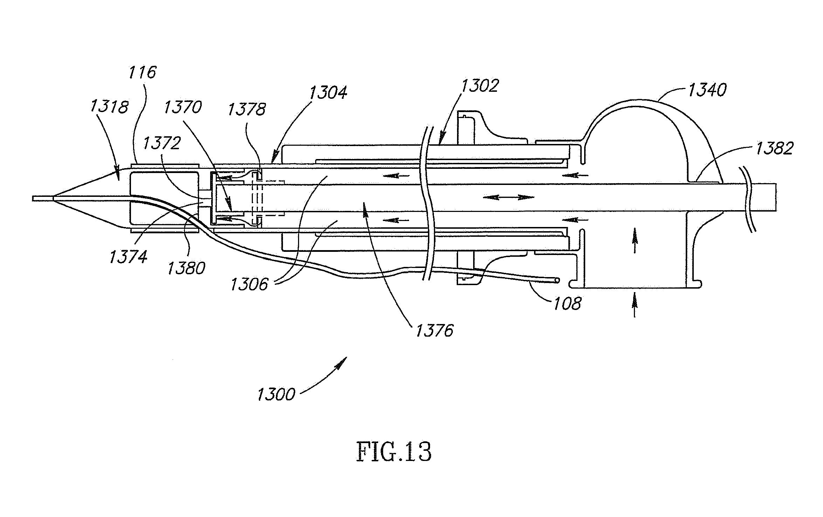

Fig. 13 shows a catheter system 1300, with a single lumen 1306, in which a mechanically actuated valve 1370 is provided, to selectively allow inflation of a balloon section 1318 thereof, in accordance with an exemplary embodiment of the invention. In general, catheter 1300 is similar to catheter 800 (Fig. 8A), in that increase of fluid pressure in lumen 1306 of an inner tube 1304 causes inner tube 1304 to advance relative to an outer tube 1302.

Unlike the embodiment shown in Fig. 8A, however, valve 1370 is provided to prevent inadvertent entry of fluid into balloon 1318. In the embodiment shown, a gasket section 1372 of valve 1370 selectively prevents flow of fluid into a port 1374 of balloon 1318. Optionally, port 1374 is an aperture in a base of balloon 1318, which also acts as a limiter 1380 for forward motion of gasket 1372. Once advancing of tube 1304 is competed, a wire 1376 attached to gasket 1372 is pulled back, unsealing port 1374. Gasket 1372 is optionally attached to limiter 1380 (not as shown), for example to limit retraction of gasket 1372.

When the valve is opened, increase of fluid pressure in lumen 1306 will cause balloon 1318 to expand. Inner tube 1304 is optionally fully advanced or locked to outer tube 1302, to prevent its further extension, for example, by attaching a brake on its outside section or providing a separate stop wire, as in Fig. 4C. A back motion limiter 1378 is optionally provided to prevent too far a retraction of gasket 1372.

Optionally, when gasket 1372 reaches back limiter 1378, further fluid flow in lumen 1306 is blocked and/or may be used to retract balloon 1318. Alternatively, gasket 1372 may be floppy or the stop apertured, so that such flow is not stopped. Optionally, this is useful when retracting tube 1304 and/or catheter 1300, to prevent fluid from lumen 1306 from entering balloon 1318 and re-inflating it.

Optionally, wire 1376 can be retracted against limiter 1380 to pull back balloon 1318 and/or tube 1304, for example past an obstruction, or a small distance. Optionally, wire 1376 is provided through a wire port 1382 in a base section 1340 of catheter 1300. For example the port design of Fig. 4C may be used, in which increase fluid pressure enhances the seal.

It should be noted that this design of valve 1370 operates automatically in that unless wire 1376 is purposely manipulated, valve 1370 will close upon fluid pressure increase, blocking further inflation of the balloon.

When an obstruction is reached in advancing tube 1304 (e.g., a narrowing in the blood vessel or narrowing 106), sudden impulses of pressure may be provided. Automatic closing of valve 1370 optionally prevents inadvertent inflation of balloon 1318.

Alternatively or additionally, wire 1376 is used to apply force to balloon 1318, helping advance it past obstructions, or for advancing balloon 1318 a small (e.g., 10 mm) or large amount (e.g. 100 mm). Alternatively or additionally, wire 1376 is used to vibrate balloon 1318, for example, to help go past obstructions. Thus, wire 1376 may be used for application of mechanical force to a forward section of catheter 1300. Optionally, balloon 1318 is slightly inflated to help widen a narrowing it is pushed past.

In some embodiments of the invention wire 1376 is used for extending tube 1304, or if only a single tube is provided, catheter 1300, instead of a fluid column in lumen 1306.

Optionally, wire 1376 is curved and is used for navigation (e.g., to turn catheter 1318).

### Alternative back limiter

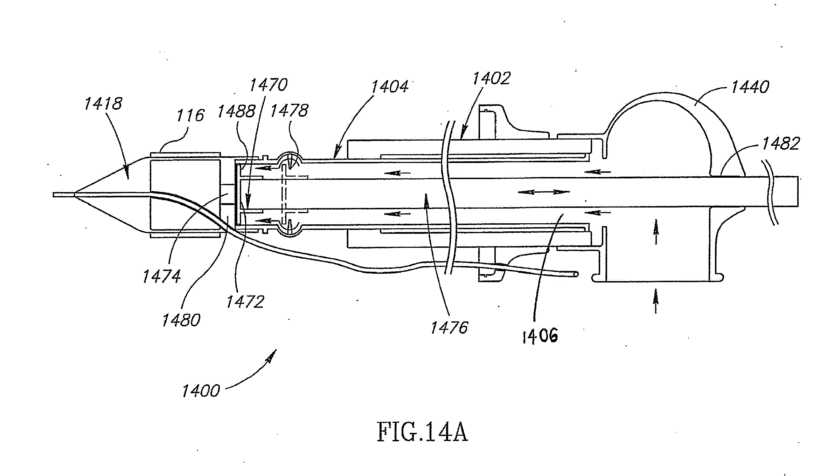

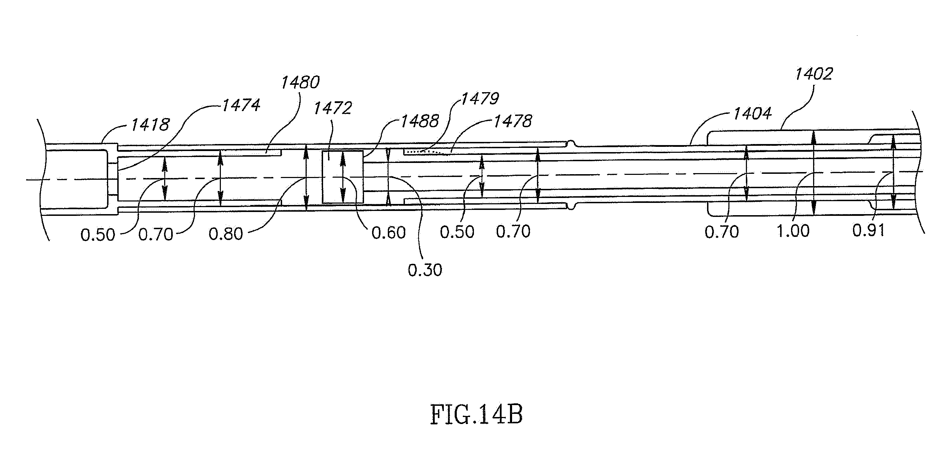

Figs. 14A and 14B show a catheter system 1400, similar to catheter 1300, with a different back limiter 1478 design in a valve 1470, in accordance with an exemplary embodiment of the invention. Fig. 14A is more schematic, while Fig. 14B is a generally to scale version of a particular implementation of the catheter of Fig. 14A.

Unlike limiter 1378 of Fig. 13, limiter 1478 is an annular ring with slots formed along its inner circumference, such that a diameter at the slotted sections is greater than a diameter of a gasket 1472 and the diameter at the non-slotted portions is smaller than the diameter of gasket 1472. Thus, even with gasket 1472 pulled back against limiter 1478, fluid flow past back limiter 1478 is possible. Optionally, the diameter of a lumen 1406 of an inner tube 1404 at limiter 1478 is increased to be greater than that of gasket 1472.

In an alternative embodiment, a back section 1488 of gasket 1472 is slotted and cone shaped and a plain ring may be used for limiter 1478. In this embodiment the maximal diameter of gasket 1472 is smaller than an inner diameter of an inner tube 1402 at the area of limiter 1478. If port 1474 is smaller in diameter than lumen 1406, gasket 1472 optionally has a diameter substantially smaller than that of lumen 1406.

It should be noted that pulling back or holding a wire 1476 of valve 1470 can be used to prevent advance of inner tube 1404 while a balloon 1418 thereof is inflated.

It should be noted that a valve as described herein may be used for other purposes, for example allowing selective sampling of blood and/or blood pressure in the body, or injection of pharmaceuticals or contrast agents. Such fluids can enter or leave (in this or in other embodiments) via the lumen which would otherwise be used to pressure balloon 1418.

Fig. 14B is a cross-sectional view generally to scale of an implementation of catheter 1400. It shows an implementation, in which back limiter 1478 is an elongate tube with axially elongate slots 1479.

### Rotating valve

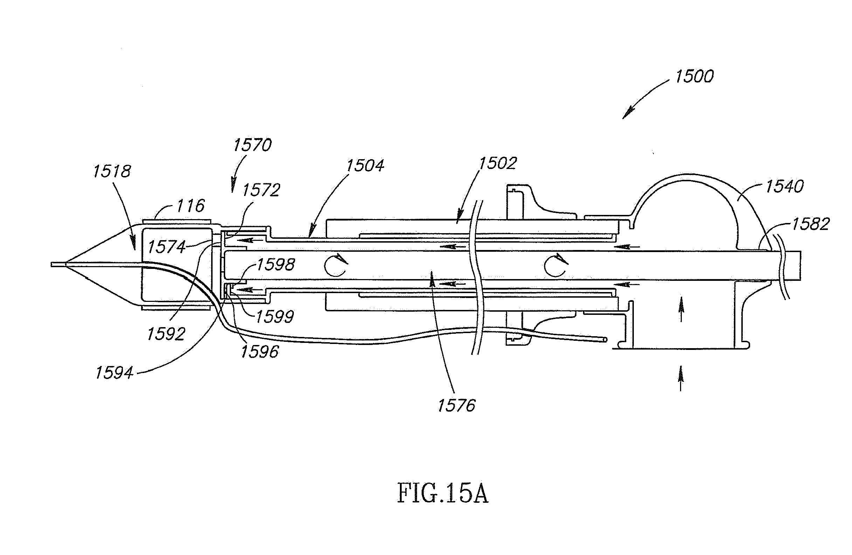

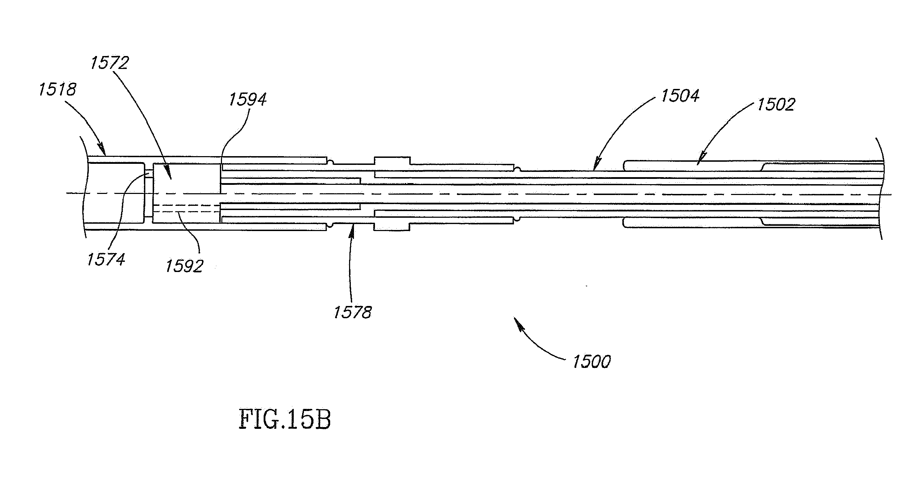

Figs. 15A and 15B show a catheter system 1500, similar to that of Fig. 13, with a rotating valve 1570 design, in accordance with an exemplary embodiment of the invention. Again, Fig. 15A shows an schematic diagram and Fig. 15B shows an implementation generally to scale.

In catheter 1500, a gasket 1572 is selectively rotated by a wire 1576, so that an aperture 1592 therein selectively matches a balloon port 1574, both optionally of axis. Optionally, gasket 1572 sits in an arcuate slot 1594, which prevents retrograde motion thereof. Slot 1594 is optionally missing adjacent balloon port 1574. Optionally, a rotation stop 1596, for example a peg that extends proximally axially past slot 1594, is provided on gasket 1572, to allow identification of its opening state and/or to simplify usage. For example, a maximal clockwise rotation will indicate (or cause) an open valve, and a maximal counter-clockwise rotation will cause a closed valve. Alternatively or additionally, a slot 1598 is formed on gasket 1572, and extends only part of an arc and rides on a peg or arcuate section 1599 which is coupled to a non-moving part of valve 1570.

### Inner and outer motion

In the embodiments described herein, both motion of the inner tube and motion of the outer tube, are provided. Potential advantages of inner tube motion include less friction against an enclosing tube/body lumen, smaller diameter and use of the inner tube for balloon inflation as well as extension. Potential advantages of outer tube motion include, increased force at tip and better sealing against leakage. As can be appreciated, the designs shown herein may be varied to have inner or outer tube motion.

Similarly there are advantages and disadvantages to the variations of having working fluid inside the inner tube or between the tubes. One consideration is sealing. Another is distance of application of force from the catheter tip. Another is effective hydraulic cross-section.

It should be noted that leakage of fluid past a seal (or if no seal is provided) may have the advantage of acting as a pressure release valve, which prevents over-pressuring. In some implementations, high pressures may degrade the controllability, possibly causing freezing or jumping. Leakage reduces these pressures, if inadvertently achieved, and increases controllability. In other embodiments, such a leak is used to provide the working fluid, possibly including a pharmaceutical, to a desired area. In should be noted that leakage of saline fluid is generally not a physiological problem.

### Outside inflation tube

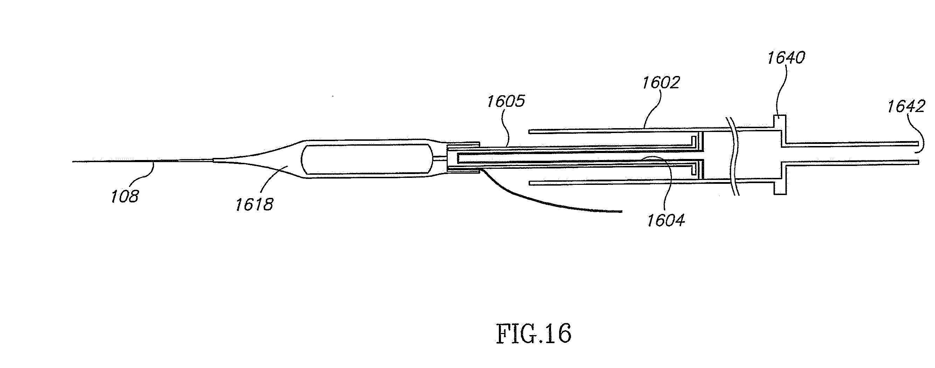

Fig. 16 shows a catheter system 1600, in which a balloon inflation tube 1605 is external to an inner tube 1604. In use, pressure is provided through a port 1642 to a base 1640, causing inner tube 1604 to advance relative to an outer tube 1602. Once advancing is completed, inner tube 1604 is removed and inflation tube 1605 is used for inflating of a balloon 1618.

### Tools

The above description has focused on balloons as a tool which is provided using the catheter. Other tools maybe provided in addition to or instead of a balloon. In one example, the lumen is used for providing other fluids, such as contrast material or a pharmaceutical. Alternatively or additionally, the balloon is a sweating balloon for providing such pharmaceuticals. Alternatively or additionally, the balloon is used to provide radioactive treatment.

Alternatively or additionally, other tools are provided, for example, an aneurysm treatment coil, a basket, RF ablation (e.g., using the lumen for wires), a drill, forceps, a closure devices (e.g., for septal defects). In an exemplary embodiment of the invention, a coil is pushed out of the lumen using an increased pressure in the lumen. In another example, a pull wire in the lumen is used to deploy a basket. This, it can be seen that in some embodiments of the invention the working fluid used to advance the balloon and/or tubes used for carrying this fluid, may perform double duty in activating a tool.

### Testing

Optionally, one or more of the following tests are applied during or after manufacture, to some or all of the catheters:
(a) determination if movement profile (e.g., in response to force and/or volume) is uniform;
(b) detecting leaks; and
(c) measuring friction (static and/or dynamic), at one or more locations along the catheter.

Optionally, some testing is done outside the body before use, for example making sure that the catheter works (e.g., test extending), checking for leaks and/or familiarizing a physician with the catheters.

In filling the catheter with fluid, a priming step is optionally performed in which fluid is dripped into the catheter, while the hub is held up and the balloon is down. Optionally vibration is used to reduce air bubbles. Optionally, the catheter is stored in vacuum.

While the above catheter system has been described in general for any type of blood vessel, it should be appreciated that particular modifications may be desired for certain vessel types. For example, different coronary vessels have different diameters and distances from the aorta, thus suggesting different catheter lengths, stiffnesses and diameters.

Measurements are provided to serve only as exemplary measurements for particular cases. The exact measurements stated in the text may vary depending on the application, the type of vessel (e.g., artery, vein, xenograft, synthetic graft), number of turns, distance of treatment area from a major blood vessel, type of tool, and/or diameter of vessels involved (e.g., 1mm, 2mm, 3mm, 5mm, aorta sized).

While the term "tube" and other geometrical shapes have been described and used for generality, it should be appreciated that this tube need not have a full body nor have a circular cross-section, in some embodiments.

It will be appreciated that the above described methods of catheter advancing and extending may be varied in many ways, including, changing the order of steps and the types of tools used. In addition, a multiplicity of various features, both of method and of devices have been described. In some embodiments mainly methods are described, however, also apparatus adapted for performing the methods are considered to be within the scope of the invention. It should be appreciated that different features may be combined in different ways. In particular, not all the features shown above in a particular embodiment are necessary in every similar embodiment of the invention. For example, the following features can be used in various embodiments: seal types, stop types, offsets, location of working fluid, balloon lumen storage, pressure-based control, extension controllers and valves. Further, combinations of the above described features, also for different embodiments, are also considered to be within the scope of some embodiments of the invention. Also within the scope of the invention are surgical kits which include sets of medical devices suitable for performing, for example, a single or a small number of catheter based procedures. In some embodiments, one or more of the devices, generally sterilize, described above, are packaged and/or sold with an instruction leaflet, describing the device dimensions and/or situations for which the device should be applied. Section headings where are provided are intended for aiding navigation and should not be construed to limiting the description to the headings. When used in the following claims, the terms "comprises", "includes", "have " and their conjugates mean "including but not limited to".

It will be appreciated by a person skilled in the art that the present invention is not limited by what has thus far been described. Rather, the scope of the present invention is limited only by the following claims.

## Claims

1. A catheter for use in a blood vessel, comprising:
an elongate body having an axis, a lumen along said axis, a proximal opening at one end, connected to the lumen and a front tip at a distal end of the body; and
an elongate hydraulic fluid column in said lumen and adapted to apply a pushing force to said front tip in a distal direction, said force being applied at an application point and wherein when pushing force is applied to said tip said tip pulls along a portion of said catheter, said portion having a length of at least 5 times a diameter of the catheter.

2. A catheter according to claim 1, wherein said application point is nearer said front tip than said proximal opening.

3. A catheter according to claim 1 or claim 2, wherein said proximal opening is adapted to be outside a human body, when the catheter is in use.

4. A catheter according to any of claims 1-3, wherein said catheter is configured so that said liquid material does not drain into said blood vessel.

5. A catheter according to any of claims 1-4, wherein said column is adapted to be advanced from outside a body.

6. A catheter according to any of claims 1-5, wherein said body comprises a collapsed tube which extends from said tip to outside of said body and which said pushing force extends collapsed tube.

7. A catheter according to any of claims 1-6, wherein said application point is less than 500 mm from a most distal point of said catheter.

8. A catheter according to any of claims 1-6, wherein said application point is less than 350 mm from a most distal point of said catheter.

9. A catheter according to any of claims 1-6, wherein said application point is less than 70 mm from a most distal point of said catheter.

10. A catheter according to any of claims 1-6, comprising an offset element between said application point and said tip, which offset element conveys said force from said column towards said tip.

11. A catheter according to any of claims 1-10, comprising a push wire adapted to apply a second force to said tip.

12. A catheter according to claim 11, wherein said push wire applies said second force at an axial position substantially the same as the axial position of said application point.

13. A catheter according to claim 11, comprising a controller configured to allow a short advance of said wire, suitable for passing a narrowing in a blood vessel.

14. A catheter according to any of claims 1-13, comprising a base hub adapted to remain outside a human body, when the catheter is in use.

15. A catheter according to claim 14, wherein said base hub has only a single port for liquid pressure.

16. A catheter according to claim 14, wherein said base hub has a plurality of ports for liquid pressure.

17. A catheter according to claim 16, wherein at least one of said ports has a cover adapted to remain closed when fluid inside said port is at 5 atmospheres of pressure or more.

18. A catheter according to claim 14, wherein said base hub comprises a pressure release valve.

19. A catheter according to claim 14, wherein said base hub comprises a port for a guide wire.

20. A catheter according to claim 14, wherein said base hub comprises a port for a pushing wire.

21. A catheter according to claim 14, wherein said base hub comprises a port for a valve control wire.

22. A catheter according to claim 14, wherein said base hub comprises a port for an extension restricting wire.

23. A catheter according to claim 22, wherein said port is configured to lock said wire when said base is pressurized above a pre-set pressure value.

24. A catheter according to claim 14, wherein said base hub comprises a selector configured for selecting to which of a plurality of lumens fluid pressure in the catheter will be coupled.

25. A catheter according to claim 14, wherein said base hub comprises a closable opening suitable for selectable user access to a lumen of the catheter through the closable opening.

26. A catheter according to claim 25, wherein said closable opening is adapted to be quickly opened by hand.

27. A catheter according to claim 14, wherein said base hub includes a catheter storage section having a length, wherein said length is less than 80% of a length of a catheter section stored therein.

28. A catheter according to any one of claims 1 to 27, comprising a base hub in fluid communication with the lumen through the proximal opening.

29. An extendible catheter comprising:
a base section adapted to remain outside a human body, when the catheter is in use;
an elongate body having a lumen and a distal tip and including a collapsed section stored in said base section; and
a liquid column adapted to apply force to said body adjacent said tip.

30. A catheter with a mechanically activated fluid valve, comprising:
an elongate body having a lumen, said lumen defining a fluid path;
a tool activated by said fluid and situated at a distal section of said elongate body;
a fluid valve at said distal section adapted to selectively convey fluid to said tool; and
a mechanical actuator coupled to said valve and extending outside of said body to control said valve.

31. A catheter for use in a blood vessel, comprising:
an elongate body having an axis, a lumen along said axis, a proximal opening at one end, connected to the lumen and a front tip at a distal end of the body; and
a wire in said lumen, said wire being adapted to apply a pushing force to said front tip in a distal direction, said force being applied at an application point.
